# EUROPÄISCHE PATENTSCHRIFT

(11) **EP 1 796 559 B1**
(45) Veröffentlichungstag und Bekanntmachung des Hinweises auf die Patenterteilung: **17.09.2008**
(21) Anmeldenummer: 05795651.8
(22) Anmeldetag: 27.09.2005
(51) Int. Cl.: A61B 17/32, A61B 17/28

(54) **CHIRURGISCHES INSTRUMENT**
SURGICAL INSTRUMENT
INSTRUMENT CHIRURGICAL

(30) Priorität: 01.10.2004 DE 102004049243
(43) Veröffentlichungstag der Anmeldung: 20.06.2007
(73) Patentinhaber: Raus, Laura, 00185 Roma (IT)
(72) Erfinder: RAUS, Laura, I-00185 Roma (IT); CAROLI, Fabrizio, I-00185 Roma (IT); SCHULZ, Peter, 79843 Löffingen (DE); LUTZE, Theodor, 78582 Balgheim (DE); WEISSHAUPT, Dieter, 78194 Immendingen (DE); NESPER, Markus, 78532 Tuttlingen (DE); FAULHABER, Konstantin, 78665 Frittlingen (DE)
(74) Vertreter: Papa, Elisabetta
(86) Internationale Anmeldenummer: PCT/EP2005/010395
(87) Internationale Veröffentlichungsnummer: WO 2006/037510

(56) Entgegenhaltungen:
- WO-A-20/04086988
- DE-A- 3 729 513
- US-A- 4 522 206
- US-A- 4 530 357
- US-A- 5 273 519
- US-A1- 2003 216 740
- US-A1- 2003 225 411

## Beschreibung

Die vorliegende Erfindung betrifft ein chirurgisches Instrument zum Entfernen von Knochen, Knorpel oder derartigem Gewebe, mit einem sich in einer Längsrichtung erstreckenden Schaft, welcher an seinem distalen Ende eine quer zur Längsrichtung oder relativ zu dieser geneigten Leitplatte trägt, mit einem am Schaft verschiebbar gelagerten Schneidelement, welches an seinem distalen Ende eine in Richtung auf die Schneidplatte weisende, zum Schneiden von Gewebe an die Schneidplatte heranführbare Schneide trägt.

In der Chirurgie kommen häufig in Form von Knochenstanzen ausgebildete chirurgische Instrumente der eingangs beschriebenen Art zum Einsatz. Je nach Größe des zu entfernenden Gewebeteil wird auf unterschiedliche Instrumente mit entsprechend dimensionierten Schäften und Schneidelementen zurückgegriffen. Es müssen daher für einen chirurgischen Eingriff mehrere Instrumente bereitgestellt werden. Ferner kann es vorkommen, daß das Instrument im Bereich zwischen Schneide und Schneidplatte im Verlauf eines chirurgischen Eingriffs verschmutzt und gereinigt werden muß. Ein Operateur muß dann das Instrument beiseite legen und mit einem anderen Instrument weiterarbeiten.

Die zweiteilige Form des Anspruchs 1 basiert sich auf der Offenbarung des US5273519.

Es ist daher Aufgabe der vorliegenden Erfindung, ein chirurgisches Instrument der eingangs beschriebenen Art so zu verbessern, das ein Operateur schnell, einfach und sicher zwischen unterschiedlichen Instrumenten wechseln kann.

Diese Aufgabe wird bei einem chirurgischen Instrument der eingangs beschriebenen Art erfindungsgemäß dadurch gelöst, das am proximalen Ende des Schafts ein erstes Kupplungselement angeordnet ist zum lösbaren, formschlüßigen Verbinden mit einer Antriebsvorrichtung, welche ein mit dem ersten Kupplungselement zusammenwirkendes zweites Kupplungselement aufweist, und daß das erste Kupplungselement ein Vielkant ist, dessen Außenflächen von einer Längsachse des Schafts radial nach außen weisen.

Die erfindungsgemäße Weiterbildung bekannter chirurgischer Instrumente der eingangs beschriebenen Art ermöglicht eine Trennung des Instruments von der Antriebsvorrichtung, so daß nur eine einzige Antriebsvorrichtung für unterschiedliche Instrumente erforderlich ist. Dies gestattet es beispielsweise, daß ein Operateur die Antriebsvorrichtung weiter in seiner Hand hält, während eine ihm assistierende Person das Instrument von der Antriebsvorrichtung löst und ein anderes Instrument, welches der Operateur benötigt, mit der Antriebsvorrichtung verbindet. Auch läßt sich so einfach und sicher ein verschmutztes Instrument von der Antriebsvorrichtung lösen und durch ein sauberes ersetzen. Durch die Ausbildung des ersten Kupplungselements als Vielkant ist eine schnelle und sichere Verbindung des Instruments mit der Antriebsvorrichtung möglich. Insbesondere kann so eine verdrehsichere und exakte Positionierung des Instruments an der Antriebsvorrichtung realisiert werden. Auch ist nicht zwingend eine Verschraubung zur Sicherung des Instruments an der Antriebsvorrichtung erforderlich, so daß das Instrument einfach und schnell von der Antriebsvorrichtung gelöst und wieder mit dieser verbunden werden kann. Die Ausgestaltung des ersten Kupplungselements in Form eines Vielkants stellt auch sicher, daß nur in Verbindung mit der Antriebsvorrichtung zugelassene Instrumente mit derselben verbunden werden können. Ferner erlaubt es insbesondere ein symmetrisch ausgebildeter Vielkant, daß das Instrument relativ zu der Antriebsvorrichtung in einer einer entsprechend der Zahl der Außenflächen des Vielkants Vielzahl von unterschiedlichen Drehstellungen angeordnet und verbunden werden kann. So lassen sich diskrete Positionen des Instruments relativ zur Antriebsvorrichtung exakt vorgeben.

Besonders einfach wird der Aufbau des Instruments, wenn der Vielkant ein Vierkant, ein Sechskant oder ein Achtkant ist. Ein Vierkant läßt sich beispielsweise in vier verschiedenen Winkelpositionen relativ zur Antriebsvorrichtung mit dieser drehsicher verbinden, wobei die Winkelpositionen jeweils um 90° zueinander verdreht sind. Entsprechend können bei einem Sechskant sechs diskrete, jeweils um 60° gegeneinander gedrehte Winkelstellungen des Instruments relativ zur Antriebsvorrichtung vorgegeben werden. Bei einem Achtkant können insgesamt acht verschiedene, gegeneinander jeweils um 45° gedrehte Winkelstellungen des Instruments vorgegeben werden, nämlich ausgehend von einer 0°-Stellung eine 45°, 90°, 135°, 180°, 225°, 270° und 315°-Stellung.

Der Aufbau des Instruments wird insgesamt vereinfacht und eine Verbindbarkeit des Instruments mit der Antriebsvorrichtung erleichtert, wenn das proximale Ende des Schafts eine zur Längsrichtung parallele Symmetrieachse aufweist.

Vorteilhaft ist es, wenn der Schaft an seinem proximalen Ende hülsenartig geformt ist und wenn das Schneidelement das hülsenartige Ende des Schafts durchsetzt. So kann auf einfache Weise eine Führung für das Schneidelement am Schaft realisiert werden.

Um auf einfache Weise eine axiale Festlegung des Schafts an der Antriebsvorrichtung zu verwirklichen, ist es günstig, wenn an das erste Kupplungselement anschließende, in Längsrichtung des Schafts wirkende Anschläge vorgesehen sind.

Gemäß einer bevorzugten Ausführungsform der Erfindung kann vorgesehen sein, daß ein in distaler Richtung wirkender Anschlag proximalseitig des ersten Kupplungselements angeordnet ist und daß ein in proximaler Richtung wirkender Anschlag distalseitig des ersten Kupplungselements angeordnet ist. Eine solche Ausgestaltung erlaubt es, daß das zweite Kupplungselement zwischen die beiden Anschläge in Richtung auf das erste Kupplungselement hin einführbar ist.

Vorteilhaft ist es, wenn das proximale Ende des Schafts eine radial nach außen geöffnete, sich in Umfangsrichtung erstreckende Nut mit einem Nutboden und in distaler und proximaler Richtung weisenden Nutseitenwänden aufweist und daß der Nutboden das erste Kupplungselement bildet. Insbesondere können die Nutseitenwände als in distaler und proximaler Richtung wirkende Anschläge dienen. Ferner ergibt sich so ein besonders einfacher Aufbau des Instruments.

Günstig ist es, wenn das proximale Ende des Schafts mindestens eine sich in radialer Richtung erstreckende Durchbrechung aufweist, wenn das Schneidelement mindestens eine in radialer Richtung weisende Vertiefung aufweist und wenn in einer geöffneten Stellung des Instruments, in welcher die Schneide von der Schneidplatte beabstandet ist, die Durchbrechung und die Vertiefung einander mindestens teilweise überlappen. Es läßt sich so eine geöffnete Stellung des Instruments sichern, beispielsweise durch Einführen eines Sicherungselements durch die Durchbrechung hindurch in die Vertiefung. Dadurch kann verhindert werden, daß bei einer Betätigung der Antriebsvorrichtung das Instrument in eine geschlossene Stellung überführt werden kann, in welcher die Schneide an der Schneidplatte anliegt. Insbesondere zu Reinigungszwecken ist eine derartige Sicherung oder Verriegelung der geöffneten Stellung des Instruments vorteilhaft, da so Verletzungen einer das Instrument reinigenden Person ausgeschlossen werden können.

Der Aufbau des Instruments wird besonders einfach, wenn die Durchbrechung eine Bohrung und die Vertiefung eine Sacklochbohrung, ein sacklochartiges Langloch oder eine Ringnut sind.

Um eine definierte Verriegelung oder Sicherung des Instruments zu erreichen, ist es günstig, wenn ein Durchmesser der Bohrung und eine Breite der Ringnut oder ein Durchmesser der Sacklochbohrung übereinstimmen oder im wesentlichen übereinstimmen und wenn die Bohrung und die Ringnut oder die Sacklochbohrung in der geöffneten Stellung in Deckung bringbar sind.

Vorteilhafterweise ist die Durchbrechung benachbart dem ersten Kupplungselement angeordnet. Eine derartige Anordnung gestattet es, daß ein Operateur die Antriebsvorrichtung hält, während ein zweite Person das Instrument in der geöffneten Stellung sichert und das Instrument in der gesicherten Stellung von der Antriebsvorrichtung löst oder den distalen Bereich des Instruments zwischen Schneidplatte und Schneide reinigt. Eine versehentliche Betätigung der Antriebsvorrichtung durch den Operateur kann daher nicht dazu führen, daß die Schneide gegen die Schneidplatte bewegt wird und eine das Instrument austauschende oder reinigende Person verletzt werden kann.

Damit die Durchbrechung für eine dem Operateur assistierende Person leicht zugänglich ist, ist es günstig, wenn die Durchbrechung distalseitig des ersten Kupplungselements angeordnet ist.

Um den Aufbau des Instruments weiter zu vereinfachen und insbesondere eine Verbindung mit der Antriebsvorrichtung auf einfache Weise zu realisieren, ist es vorteilhaft, wenn das Schneidelement an seinem proximalen Ende ein drittes, mit einem Antriebselement der Antriebsvorrichtung lösbar verbindbares Kupplungselement trägt und wenn sich an das dritte Kupplungselement in Längsrichtung des Schafts wirkende Anschläge anschließen. Insbesondere ermöglicht eine derartige Ausgestaltung, daß das Antriebselement, beispielsweise ein Mitnehmer der Antriebsvorrichtung, zwischen die Anschläge eingreift oder diese umgreift, um eine Antriebskraft der Antriebsvorrichtung sowohl in proximaler als auch in distaler Richtung parallel zur Längsrichtung auf das Schneidelement zu übertragen.

Günstig ist es, wenn ein in distaler Richtung wirkender Anschlag proximalseitig des dritten Kupplungselements angeordnet ist und wenn ein in proximaler Richtung wirkender Anschlag distalseitig des ersten Kupplungselements angeordnet ist. Es vereinfacht sich so eine Betätigung des Schneidelements, denn ein Mitnehmer kann auf diese Weise zwischen die beiden Anschläge eintauchen.

Gemäß einer weiteren bevorzugten Ausführungsform der Erfindung kann vorgesehen sein, daß ein in distaler Richtung wirkender Anschlag distalseitig des dritten Kupplungselements angeordnet ist und daß ein in proximaler Richtung wirkender Anschlag proximalseitig des ersten Kupplungselements angeordnet ist. Beispielsweise kann das Antriebselement so das dritte Kupplungselement distalseitig und proximalseitig umgreifen. Es ist jedoch auch denkbar, daß ein als Mitnehmer ausgebildetes drittes Kupplungselement zwischen zwei in distaler und proximaler Richtung wirkende Anschläge des Antriebselements eintaucht.

Grundsätzlich wäre es denkbar und auch vorteilhaft, wenn das dritte Kupplungselement zylindrisch ausgebildet ist und einen runden Querschnitt aufweist. Ebenso könnte es auch einen elliptischen Querschnitt aufweisen. Der Aufbau des Instruments vereinfacht sich allerdings, wenn das dritte Kupplungselement ein Vielkant ist. Insbesondere ist es günstig, wenn das dritte Kupplungselement zum ersten Kupplungselement ähnlich ausgebildet ist.

Um Gewebe in definierter Weise abtrennen zu können, ist es günstig, wenn die Schneide eine in sich geschlossene ringförmige Schneide ist. Beispielsweise kann sie kreisförmig oder auch viereckig ausgebildet sein.

Um zu verhindern, daß sich mit dem Instrument entferntes Gewebe im Bereich eines Operationssitus in unerwünschter Weise verteilt, ist es vorteilhaft, wenn das Schneidelement eine sich ausgehend von seinem distalen Ende in proximaler Richtung erstreckende Schneidgutausnehmung zum Aufnehmen von entferntem Gewebe aufweist und wenn die Schneide eine Öffnung der Schneidgutausnehmung mindestens teilweise umgibt. Wird mit der Schneide durch Heranführen an die Schneidplatte Gewebe entfernt, so wird dies durch die erfindungsgemäße Ausgestaltung direkt in die Schneidgutausnehmung hineinbewegt und darin gespeichert. Die Schneidgutausnehmung dient somit als eine Art Gewebe oder Knochenspeicher für entferntes Gewebe- oder entfernten Knochen.

Gemäß einer weiteren bevorzugten Ausführungsform der Erfindung kann vorgesehen sein, daß die Schneidgutausnehmung sacklochartig ausgebildet ist und daß ein in Längsrichtung am Schneidelement verschiebbarer Auswerfer vorgesehen ist, welcher von einer zurückgezogenen Stellung, in welcher er die Schneidgutausnehmung freigibt, in eine Auswurfstellung, in welcher er die Schneidgutausnehmung vollständig oder im wesentlichen vollständig ausfüllt, verschiebbar ist und umgekehrt. Mit dem Auswerfer kann die Schneidgutausnehmung einfach und sicher entleert werden, wenn sie teilweise oder vollständig mit entferntem Gewebe gefüllt ist. Dabei ist es denkbar, daß der Auswerfer von der Antriebsvorrichtung betätigbar ist oder am Instrument selber betätigt werden kann. Durch Bewegung des Auswerfers können entferntes Gewebe oder Knochen in distaler Richtung bewegt und durch die Öffnung der Schneidgutausnehmung wieder aus dieser herausgeschoben werden.

Auf einen Auswerfer kann insbesondere dann verzichtet werden, wenn ein proximales Ende der Schneidgutausnehmung mit einer seitlichen Auswurföffnung versehen ist. Entferntes Gewebe wird durch die Öffnung der Schneidgutausnehmung in diese hineingeschoben, kann jedoch dann, wenn die Schneidgutausnehmung vollständig gefüllt ist, durch Nachschieben von zusätzlich entferntem Gewebe seitlich durch die Auswurföffnung herausgeschoben werden. Werden jedoch nicht alle Gewebeteile aus der Auswurföffnung wieder herausgeschoben, so kann beispielsweise durch die Auswurföffnung auch ein Auswerfer eingeführt und das in der Schneidgutausnehmung enthaltene Gewebe durch die distalseitige Öffnung herausgeschoben werden.

Damit eine Bedienperson oder eine dieser assistierende Person leicht erkennen kann, ob die Schneidgutausnehmung gefüllt ist und eventuell geleert werden muß, ist es günstig, wenn das Schneidelement im Bereich der Schneidgutausnehmung seitlich mit mindestens einer Sichtöffnung versehen ist. Je nach Ausgestaltung der Sichtöffnung kann so ein Füllstand der Schneidgutausnehmung direkt erkannt werden.

Damit durch die Sichtöffnung kein entferntes Gewebe austreten kann, ist es günstig, wenn die mindestens eine Sichtöffnung eine Breite quer zur Längsrichtung aufweist, die kleiner als der Innendurchmesser der Schneidgutausnehmung ist.

Um bei vorhandener Sichtöffnung eine maximale Stabilität des Instruments zu erreichen, ist es günstig, wenn mehrere langlochartige Sichtöffnungen vorgesehen sind.

Um zumindest zu erkennen, wenn die Schneidgutausnehmung nahezu gefüllt ist und um das Instrument maximal stabil auszubilden, ist es günstig, wenn die mindestens eine Sichtöffnung distalseitig benachbart dem proximalen Ende der Schneidgutausnehmung angeordnet ist.

Günstigerweise ist eine Führung vorgesehen zum Führen einer Bewegung des Schneidelements relativ zum Schaft in Längsrichtung. Dadurch wird die Stabilität des Instruments erhöht, insbesondere dann, wenn große Kräfte auf das Schneidelement wirken.

Um eine möglichst lange Lebensdauer des Instruments erreichen zu können, ist es vorteilhaft, wenn die Schneide und/oder die Schneidplatte mit einer abriebfesten Beschichtung versehen sind.

Eine besonders lange Standzeit des Instruments wird erreicht, wenn die abriebfeste Beschichtung Titannitrid (TiN) ist oder Titannitrid (TiN) enthält.

Gemäß einer bevorzugten Ausführungsform der Erfindung kann vorgesehen sein, daß am Schaft eine mit einer Dekodiereinheit der Antriebsvorrichtung zusammenwirkende Kodiereinheit vorgesehen ist zur Kodierung der Art oder des Typs des Schneidelements. Die Kodierung kann zur Identifikation der Art oder des Typs des Schafts dienen. Dies hat den Vorteil, daß mit der Antriebsvorrichtung nur entsprechend der Art oder des Typs des Schneidelements und/oder des Schafts zulässige Antriebskräfte auf das Schneidelement übertragen werden können. Ferner wird dadurch erreicht, daß nur für eine bestimmte Antriebsvorrichtung vorgesehene chirurgische Instrumente mit der Antriebsvorrichtung verbunden werden können.

Besonders einfach wird der Aufbau der Kodiereinheit, wenn diese mindestens einen vom Schaft abstehenden Vorsprung umfaßt. Je nach Zahl der unterschiedlichen, zu kodierenden Instrumente, können ein oder mehrere, an bestimmten Positionen des Schafts anzuordnende Vorsprünge vorgesehen sein.

Vorteilhaft ist es, wenn ein chirurgisches Instrument der eingangs beschriebenen Art oder eines der oben beschriebenen Instrumente eine mit dem Schaft und dem Schneidelement formschlüssig lösbar verbindbare Antriebsvorrichtung mit einer Antriebseinheit zum Erzeugen einer Antriebskraft in einer Antriebsrichtung vorsieht, welche ein mit dem ersten Kupplungselement zusammenwirkendes zweites Kupplungselement umfaßt. Die Antriebsvorrichtung kann manuell oder mittels eines mechanischen oder elektromechanischen Antriebs betätigt werden. Ferner können der Schaft und das Schneidelement auf einfache Weise von der Antriebsvorrichtung gelöst und wieder mit der Antriebsvorrichtung verbunden werden.

Der Aufbau des Instruments wird besonders einfach und kompakt, wenn die Antriebseinheit eine fluidbetriebene Antriebseinheit ist, wenn die Antriebsrichtung nichtparallel zur Längsrichtung ist und wenn die Antriebsvorrichtung eine Kraftumlenkeinheit aufweist, zum Umlenken der in Antriebsrichtung wirkenden Antriebskraft in eine in Längsrichtung auf das Schneidelement wirkende Betätigungskraft. Beispielsweise läßt sich so die Antriebseinheit in einen Griff der Antriebsvorrichtung integrieren, welcher seitlich oder quer zur Längsrichtung abstehend orientiert ist. Dies verkürzt eine Baulänge des Instruments mit Antriebsvorrichtung im Vergleich zu bekannten Instrumenten mit Antriebsvorrichtung.

Vorzugsweise ist die Antriebsrichtung quer oder im wesentlichen quer oder schräg zur Längsrichtung orientiert. Dies erlaubt es, die Antriebseinheit beispielsweise in einem Griffbereich der Antriebsvorrichtung anzuordnen, welcher quer oder im wesentlichen quer oder schräg zur Längsrichtung abstehend vorgesehen ist.

Günstig ist es, wenn die Antriebseinheit eine fluidbetriebene Antriebseinheit ist, wenn die Antriebsrichtung parallel seitlich versetzt zur Längsrichtung ist und wenn die Antriebsvorrichtung eine Kraftumlenkeinheit aufweist zum Umlenken der in Antriebsrichtung wirkenden Antriebskraft in eine in Längsrichtung auf das Schneidelement wirkende Betätigungskraft. Durch diese Ausgestaltung kann eine besonders schlanke Bauform des Instruments erreicht werden, da beispielsweise die mindestens einen Fluidzylinder umfassende Antriebseinheit parallel und seitlich versetzt zur Längsachse des Schafts angeordnet werden kann. Durch die Kraftumlenkeinheit kann dann die parallel zur Längsachse des Schafts von der Antriebseinheit erzeugte Antriebskraft in Richtung der Längsachse des Schneidelements umgelenkt werden.

Ganz ohne elektrische Energieversorgung kann das Instrument auskommen, wenn die Antriebseinheit mindestens einen Fluidzylinder umfaßt. Durch Beaufschlagen des Fluidzylinders mit einem Fluid kann ein Kolben des Fluidzylinders parallel zu dessen Längsachse in definierter Weise bewegt werden. So ergibt sich insgesamt ein besonders einfacher Aufbau des Instruments.

Um eine Antriebskraft in zueinander entgegengesetzten Richtungen zu ermöglichen, ist es vorteilhaft, wenn der mindestens eine Fluidzylinder ein doppeltwirkender Fluidzylinder ist. So kann auch mit einem einzigen Fluidzylinder das Schneidelement sowohl in distaler als auch in proximaler Richtung bewegt werden.

Günstigerweise ist der mindestens eine Fluidzylinder ein Pneumatik- oder Hydraulikzylinder. Ein Pneumatikzylinder kann insbesondere mit Druckluft beaufschlagt werden, um eine Antriebskraft zu realisieren. Ein Hydraulikzylinder kann vorzugsweise mit einer körperverträglichen Flüssigkeit betrieben werden.

Gemäß einer bevorzugten Ausführungsform der Erfindung kann vorgesehen sein, das die Kraftumlenkeinheit eine Über/- oder Untersetzungseinheit für die Antriebskraft umfaßt oder bildet zum Erzeugen einer Betätigungskraft, die einem einem Über/- oder Untersetzungsverhältnis der Über/- oder Untersetzungseinheit entsprechenden Vielfachen entspricht. Insbesondere bei einer vorgegebenen Baugröße der Antriebseinheit läßt sich so eine noch größere Antriebskraft auf einfache Weise erzeugen.

Der Aufbau des Instruments wird besonders einfach, wenn die Kraftumlenkeinheit einen Winkelhebel umfaßt, wenn der Winkelhebel einen ersten und einen zweiten, relativ zum ersten abgewinkelten Hebelarm aufweist, wenn der Winkelhebel im Übergangsbereich der beiden Hebelarme am relativ zum Schaft feststehenden Teil der Antriebsvorrichtung verschwenkbar gelagert ist, wenn die Antriebseinheit mit einem freien Ende des ersten Hebelarms zusammenwirkt und wenn ein freies Ende des zweiten Hebelarms mit dem Schneidelement zusammenwirkt. Durch diesen einfachen mechanischen Aufbau kann eine in Antriebsrichtung wirkende Antriebskraft in Längsrichtung des Instruments umgelenkt werden.

Besonders einfach wird eine Kraftübertragung von der Antriebseinheit auf das Schneidelement und ein Zusammenwirken derselben, wenn der zweite Hebelarm einen mit dem proximalen Ende des Schneidelements zusammenwirkenden Mitnehmer trägt.

Günstig ist es, wenn das freie Ende des zweiten Hebelarms zwischen den in distaler Richtung wirkenden, proximalseitig des dritten Kupplungselements angeordneten Anschlag und den in proximaler Richtung wirkenden, distalseitig des dritten Kupplungselements angeordneten Anschlag eintaucht. Eine Verschwenkbewegung des Winkelhebels vor und zurück kann so auf einfache Weise in eine Vor- und Zurückbewegung des Schneidelements umgesetzt werden. Vorzugsweise taucht das freie Ende des zweiten Hebelarms derart zwischen die beiden Anschläge ein, daß er seitlich nicht übersteht. Damit schließt er quer zur Längsrichtung quasi bündig ab, so daß insgesamt eine besonders kompakte Bauform im Verbindungsbereich zwischen dem Winkelhebel und dem dritten Kupplungselement erzielt wird.

Vorteilhaft kann es ferner sein, wenn das freie Ende des zweiten Hebelarms distalseitig in proximaler Richtung wirkend und proximalseitig in distaler Richtung wirkend am dritten Kupplungselement angreift. Eine solche Ausgestaltung eignet sich insbesondere dann, wenn das dritte Kupplungselement als Mitnehmer ausgebildet ist.

Die Übersetzungseinheit wird in ihrem Aufbau besonders einfach, wenn die beiden Hebelarme des Winkelhebels unterschiedlich lang sind. Kräfte können dann entsprechend der Längenverhältnisse der beiden Hebelarme übersetzt oder auch untersetzt werden.

Günstigerweise beträgt ein Verhältnis der Länge des ersten Hebelarms zur Länge des zweiten Hebelarms mindestens 2 : 1. Vorzugsweise beträgt das Längenverhältnis 3 : 1. Dadurch läßt sich Antriebskraft auf einfach Weise mindestens verdoppeln, insbesondere verdreifachen, das heißt, daß die auf das Schneidelement wirkende Betätigungskraft mindestens doppelt, vorzugsweise dreimal so groß ist wie die von der Antriebseinheit erzeugte Antriebskraft.

Der Aufbau des Instruments wird weiter vereinfacht, wenn ein freies Ende eines Kolbens des mindestens einen Fluidzylinders mit dem ersten Hebelarm gelenkig verbunden ist. Auf diese Weise wird die Zahl der erforderlichen Bauteile des Instruments minimiert. Bei unterschiedlich langen Hebelarmen dient der Winkelhebel somit gleichzeitig als Kraftumlenkeinheit und Übersetzungseinheit.

Die Handhabbarkeit des Instruments wird verbessert, wenn die Antriebsvorrichtung ein Gehäuse umfaßt, wenn ein erster Teil des Gehäuses in Form eines Griffes ausgebildet ist, wenn ein zweiter Teil des Gehäuses ausgebildet ist zur Aufnahme des proximalen Endes des Schaft und wenn die Antriebseinheit im ersten Teil des Gehäuses angeordnet ist. Ferner dient das Gehäuse dem Schutz der darin angeordneten Teile der Antriebsvorrichtung, insbesondere der Antriebseinheit.

Vorteilhaft ist es, wenn der Schaft relativ zur Antriebsvorrichtung um eine zur Längsrichtung parallele Drehachse verdrehbar und in einer beliebigen Drehstellung relativ zur Antriebsvorrichtung reversibel festlegbar ist. Je nach operativer Anforderung kann der Schaft in eine gewünschte Drehstellung gebracht und festgelegt werden, was insbesondere das über Kopf oder seitliche Arbeiten mit dem Instrument gestattet.

Um mit der Antriebseinheit unterschiedliche Kräfte aufbringen zu können, ist es günstig, wenn zwei gekoppelte, getrennt ansteuerbare Fluidzylinder vorgesehen sind. Mit zwei Fluidzylindern können insgesamt vier definierte Antriebskräfte eingestellt werden, nämlich keine, eine erste, eine zweite und eine dritte Antriebkraft, wobei die erste und zweite erzeugt wird durch Beaufschlagen jeweils eines Zylinders mit einem Fluid, die dritte durch gleichzeitige Beaufschlagung beider Zylinder mit dem Fluid.

Um insgesamt vier unterschiedliche Antriebskräfte vorgeben zu können, ist es vorteilhaft, wenn die beiden Fluidzylinder unterschiedliche Wirkungsquerschnitte aufweisen.

Vorteilhaft ist es, wenn ein Verhältnis der Wirkungsquerschnitte in einem Bereich von 4 : 1 bis 9 : 1 liegt. Eine solche Ausgestaltung ermöglicht es, das Schneidelement mit der Schneide an die Schneidplatte mit etwa 10 bis 20 Prozent der Maximalkraft, die mit der Antriebseinheit erzeugbar ist, heranzuführen. Dadurch wird verhindert, daß zu entfernendes Gewebe von der Schneide durchtrennt wird. Ein Operateur kann so das Instrument an das zu entfernende Gewebe heranführen, mit etwa 10 bis 20 Prozent der Maximalkraft der Antriebseinheit die Schneide gegen das zu entfernende Gewebe bewegen und erst nach abgeschlossener Justierung das Gewebe durch Beaufschlagen des Schneidelements mit der von der Antriebseinheit zur Verfügung stellbaren Maximalkraft beaufschlagen.

Um eine einfache Verbindung des Schafts mit der Antriebsvorrichtung zu gestatten, ist es günstig, wenn das zweite Kupplungselement an der Antriebsvorrichtung korrespondierend zum ersten Kupplungselement ausgebildet ist.

Besonders einfach wird der Aufbau des Instruments, wenn das zweite Kupplungselement zwei oder mindestens zwei vom ersten Teil des Gehäuses in Richtung auf den Schaft vorstehende Haltevorsprünge umfaßt. Die Vorsprünge können formschlüssig oder im wesentlichen formschlüssig in eine Umfangsnut des Schafts eintauchen, deren Nutboden durch den Vielkant gebildet wird.

Gemäß einer weiteren bevorzugten Ausführungsform der Erfindung kann vorgesehen sein, daß der erste Teil des Gehäuses eine Aufnahme umfaßt, in welche das proximale Ende des Schafts einbringbar ist und aus welcher das distale Ende des Schafts in einer eingelegten Stellung, in welcher er in Längsrichtung unverschieblich gehalten ist, in distaler Richtung aus dem Gehäuse hervorsteht, daß die Aufnahme eine Einlegeöffnung zum Einlegen des distalen Endes des Schafts in einer Enlegerichtung quer zur Längsrichtung aufweist und daß eine Abdeckung zum Verschließen der Einlegeöffnung und zum Sichern des Schafts in der Aufnahme vorgesehen ist. Durch diese vorteilhafte Ausgestaltung können der Schaft und das Schneidelement einfach und schnell mit der Antriebsvorrichtung verbunden werden. Insbesondere ist es hierzu nur erforderlich, die Abdeckung von der Einlegeöffnung zu entfernen, das proximale Ende des Schafts in die Aufnahme einzulegen und die Aufnahme, das heißt die Einlegeöffnung, wieder mit der Abdeckung zu verschließen. Der Schaft ist dann einerseits mit der Antriebsvorrichtung verbunden und gleichzeitig an dieser gesichert. Auf eine umständliche Verschraubung des Schafts mit der Antriebsvorrichtung kann verzichtet werden.

Ein besonders sicherer Halt des Schafts in der Aufnahme wird erreicht, indem die Abdeckung am Gehäuse verschiebbar gelagert und zum Öffnen der Einlegeöffnung in proximaler Richtung verschiebbar ist. Zum Öffnen und Schließen der Einlegeöffnung muß die Abdeckung lediglich parallel zur Längsrichtung in proximaler beziehungsweise distaler Richtung verschoben werden.

Gemäß einer weiteren bevorzugten Ausführungsform der Erfindung kann vorgesehen sein, daß die Abdeckung am Gehäuse um eine Schwenkachse verschwenkbar gelagert ist. Dadurch ist es möglich, die Einlegeöffnung durch Wegklappen der Abdeckung zu öffnen. Nach Einlegen des Schaft in die Aufnahme kann die Abdeckung wieder in ihre Ausgangsstellung zurück verschwenkt werden. Ein Verschwenken der Abdeckung kann auch mit einem Verschieben derselben kombiniert werden, beispielsweise durch Führen der Abdeckung in einer Kulisse, die zunächst nur eine Translationsbewegung des Deckels ermöglicht und erst nach einem bestimmten Verschiebeweg eine Verschwenkung. Die Verschwenkbewegung kann dabei parallel oder quer zur Längsrichtung möglich sein.

Günstig ist es, wenn die Schwenkachse parallel oder quer zur Längsrichtung verläuft. Je nach Ausgestaltung der Antriebsvorrichtung, insbesondere des Gehäuses, ist es günstig, entweder die Abdeckung um eine Schwenkachse parallel oder quer zur Längsrichtung zu verschwenken. Insbesondere dann, wenn die Abdeckung auch verschiebbar gelagert ist, ist eine Verschwenkung um eine Schwenkachse quer zur Längsrichtung vorteilhaft, da nach Öffnen der Einlegeöffnung und Verschwenken der Abdeckung die Abdeckung nicht in voller Länge vom Instrument abstehen muß, sondern nur um eine um den Verschiebeweg verkürzte Länge.

Eine besonders sichere Handhabung des Instruments ist gewährleistet, wenn bei geöffneter Abdeckung eine Energie- und/oder Betriebsmittelzufuhr der Antriebseinheit unterbrochen ist. So wird sichergestellt, daß bei geöffneter Abdeckung eine Bewegung des dritten Kupplungselements verhindert wird, die zu einer Verletzung einer Person, die den Schaft des Instruments auswechseln möchte, verhindert wird. Hierzu kann insbesondere ein mechanisches, elektrisches, elektronisches oder fluidbetätigtes Schaltelement vorgesehen sein zum Schalten eines Energie- oder Betriebsmittelflußes.

Um zu verhindern, daß beim Reinigen des Instruments die Antriebsvorrichtung betätigt werden kann, ist es vorteilhaft, wenn an der Antriebsvorrichtung ein zur Durchbrechung und zur Vertiefung korrespondierendes Sicherungselement quer zur Längsrichtung beweglich gelagert ist, welches in einer Sicherungsstellung in die Vertiefung eintaucht und in einer Lösestellung die Vertiefung freigibt. Das Sicherungselement gestattet es, daß ein Operateur die Antriebsvorrichtung hält, während eine zweite Person das Sicherungselement von der Lösestellung in die Sicherungsstellung überführt, so daß eine Betätigung der Antriebsvorrichtung durch den Operateur wirkungslos bleibt. So wird sichergestellt, daß eine Person, die den mit der Antriebsvorrichtung verbundenen Schaft austauschen oder diesen reinigen möchte, nicht durch ein versehentliches Betätigen der Antriebsvorrichtung durch den Operateur verletzt wird.

Damit das Sicherungselement für eine dem Operateur assistierende Person leicht zugänglich ist, ist günstigerweise das Sicherungselement am ersten Teil des Gehäuses benachbart dem zweiten Kupplungselement angeordnet und federnd vorgespannt in der Lösestellung gehalten. Das Instrument muß so aktiv von einer zweiten Person in die Sicherungsstellung überführt werden. Wird das Sicherungselement nicht betätigt, kann ein Operateur durch Betätigen der Antriebsvorrichtung das Instrument in gewünschter Weise zum Entfernen von Gewebe einsetzen.

Gemäß einer bevorzugten Ausführungsform der Erfindung kann vorgesehen sein, daß die Antriebsvorrichtung eine mit der Kodiereinheit zusammenwirkende Dekodiereinheit aufweist zum Dekodieren der Art oder des Typs des Schneidelements. So wird sichergestellt, daß bestimmte Schneidelemente nicht mit einer zu großen Betätigungskraft beaufschlagt werden können. Dies kann beispielsweise dadurch realisiert werden, daß die mindestens einen Vorsprung umfassende Kodiereinheit ein oder mehrere Schaltelemente mechanisch betätigt, die eine Energie und/oder Betriebsmittelzufuhr der Antriebseinheit begrenzen.

Die nachfolgende Beschreibung bevorzugter Ausführungsformen der Erfindung dient im Zusammenhang mit der Zeichnung der näheren Erläuterung. Es zeigen:
- Figur 1:: eine Längsschnittansicht durch eine Knochenstanze in einer unbetätigten Stellung;
- Figur 2:: eine Längsschnittansicht ähnlich Figur 1 in einer betätigten Stellung;
- Figur 3:: eine perspektivische Ansicht der Knochenstanze aus Figur 1 beim Austausch des Schafts;
- Figur 4:: eine vergrößerte Längsschnittansicht einer Schaftaufnahme des Instruments beim Austauschen des Schafts;
- Figur 5:: eine Querschnittsansicht längs Linie 5 - 5 in Figur 4;
- Figur 6:: eine Seitenansicht einer zweiten Ausführungsform einer Knochenstanze; und
- Figur 7:: eine Seitenansicht einer dritten Ausführungsform einer Knochenstanze.

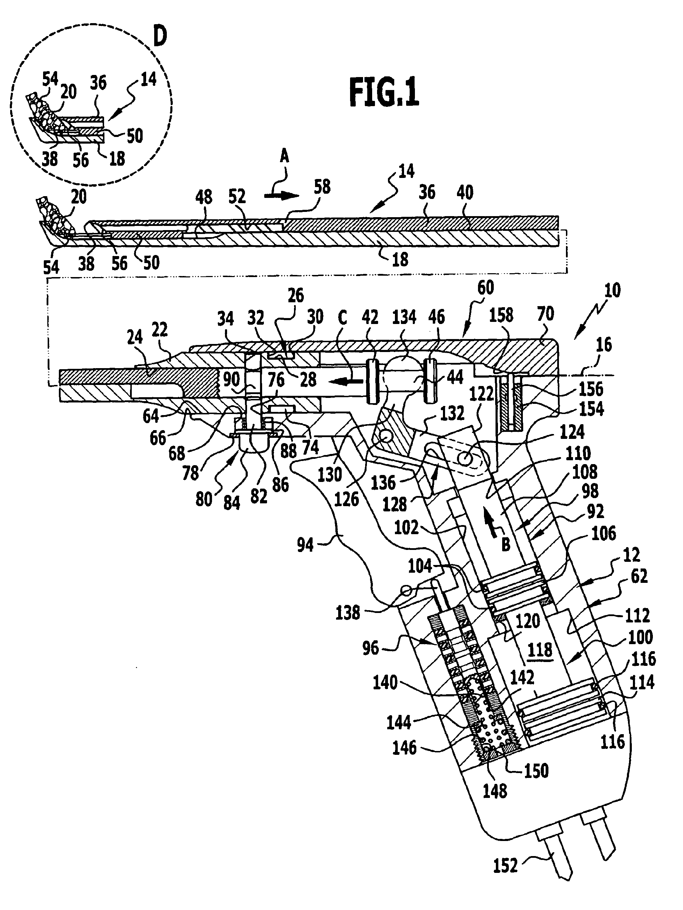

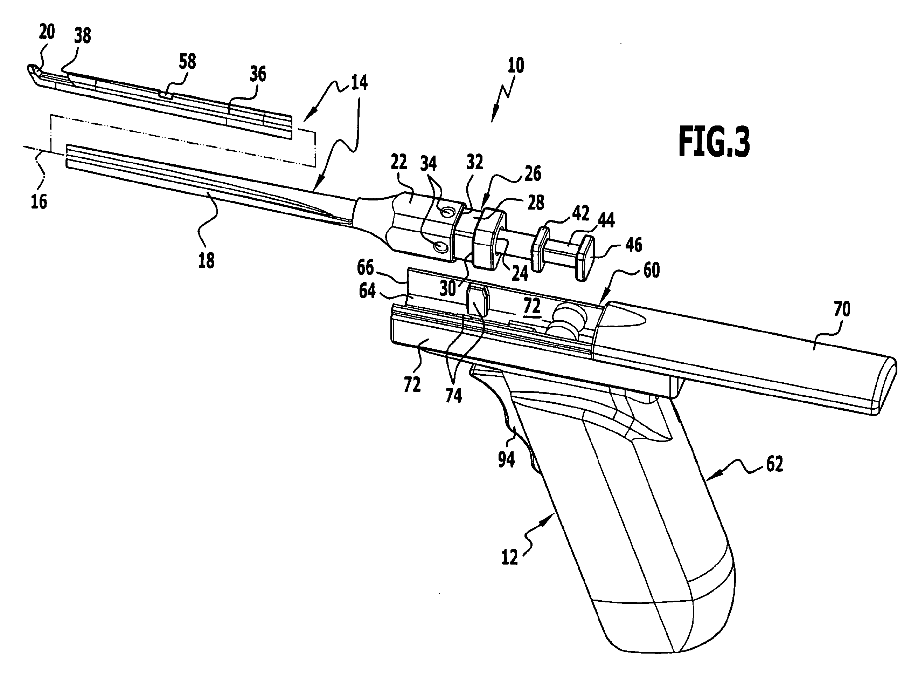

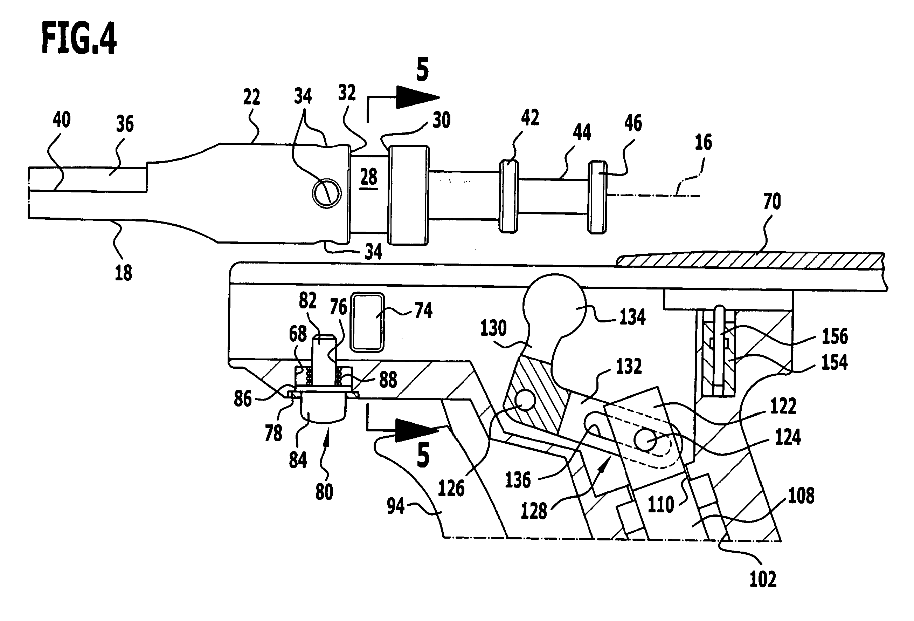

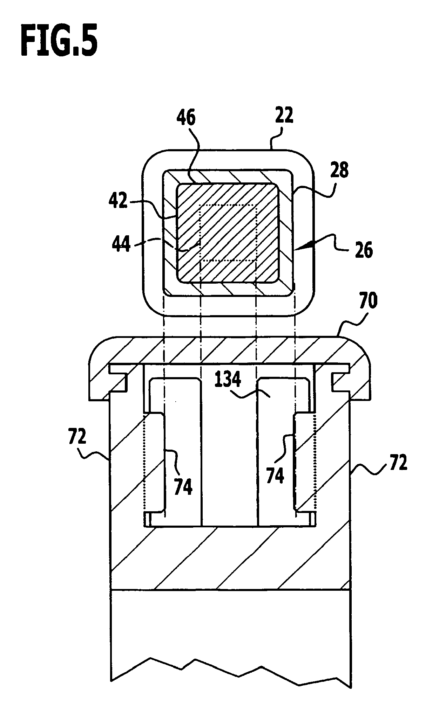

In den Figuren 1 bis 5 ist ein erfindungsgemäßes chirurgisches Instrument in Form einer Knochenstanze dargestellt, die insgesamt mit dem Bezugszeichen 10 versehen ist. Die Knochenstanze 10 umfaßt zwei wesentliche Baugruppen, nämlich ein Griffteil 12 sowie ein mit dem Bezugszeichen 14 versehenes Stanzwerkzeug.

Das Stanzwerkzeug 14 umfaßt einen sich in einer Längsrichtung 16 erstreckenden langgestreckten Schaft 18, welcher an seinem distalen Ende eine um etwa 45° gegenüber der Längsrichtung 16 geneigte Schneidplatte 20 trägt. Ein proximales Ende des Schafts bildet ein Kupplungsstück 22, welches in Form eines im Querschnitt quadratischen, langgestreckten Quaders mit angefasten Längskanten ausgebildet ist. Das Kupplungsstück 22 ist mit einer die Längsachse 16 definierenden Durchgangsbohrung 24 versehen. Des weiteren ist etwas beabstandet vom proximalen Ende des Schafts 18 am Kupplungstück 22 eine sich in Umfangsrichtung erstreckende nutartige Vertiefung 26 ausgebildet, wobei ein Boden der Vertiefung einen Vierkant 28 bildet. Er dient als erstes Kupplungselement zum verdrehsicheren Verbinden des Stanzwerkzeugs 14 mit dem Griffteil 12. Eine sich proximalseitig an den Vierkant 28 anschließende Seitenwand 30 der Vertiefung 26 bildet einen in distaler Richtung wirkenden Anschlag. Eine sich distalseitig an die Vertiefung 26 anschließende Seitenwand 32 bildet einen in proximaler Richtung wirkenden Anschlag.

Distalseitig der Vertiefung 26 sind am Kupplungsstück 22 vier identische Sicherungsbohrungen 34 ausgebildet, die sich in radialer Richtung bezogen auf die Längsachse 16 bis zur Durchgangsbohrung 24 durch das Kupplungsstück 22 hindurch erstrecken. Ferner verjüngt sich das quaderförmige Kupplungsstück 22 distalseitig im Durchmesser und weist an seinem Ende eine im wesentlichen rundhülsenartige Form auf.

Das Stanzwerkzeug 14 umfaßt eine Stanze 36, welche an ihrem distalen Ende eine gegenüber der Längsachse 16 um denselben Winkel wie die Schneidplatte 20 geneigte Schneide 38 trägt. Die Stanze 38 liegt im wesentlichen auf ihrer gesamten Länge flächig an einer Schaftfläche 40 des Schafts 18 an. Proximalseitig durchsetzt die Stanze 36 die Durchgangsbohrung 24 des Kupplungsstücks 22. In diesem Bereich ist die distalseitig im übrigen im Querschnitt im wesentlichen quaderförmig ausgebildete Stanze 36 zylindrisch geformt. An den zylindrischen Abschnitt der Stanze 36 schließt sich proximalseitig ein quadratischer, plattenförmiger Flansch 42 an, welcher distalseitig einen Kupplungsvierkant 44 begrenzt und einen in proximaler Richtung wirkenden Anschlag bildet. An den Kupplungsvierkant 44 schließt sich, ein Ende der Stanze 36 bildend, eine quadratische Abschlußplatte 46 an, die einen in distaler Richtung wirkenden Anschlag bildet. Der Flansch 42 bildet zudem einen in distaler Richtung wirkenden Anschlag, welcher am proximalen Ende des Kupplungsstücks 22 anschlägt, wenn die Schneide 38 ihre distalste Stellung einnimmt, also an der Schneidplatte 20 anliegt.

Zur Stabilisierung einer Bewegung der Stanze 36 relativ zum Schaft 18 ist zusätzlich zu der durch das Kupplungsstück 22 ausgebildeten Führung am distalen Ende Schafts eine Führungsnut 48 angeordnet, welche sich von der Schaftfläche 40 weg im Querschnitt erweitert. In der Führungsnut 48 ist ein an der Stanze 36 abstehender Führungsvorsprung 50 geführt, welcher sich parallel zur Längsachse 16 ausgehend vom distalen Ende der Stanze 36 in proximaler Richtung erstreckt. Der Führungsvorsprung 50 und die Führungsnut 48 sind im wesentlichen korrespondierend ausgebildet, beispielsweise können sie eine Schwalbenschwanzform aufweisen.

Die Stanze 36 ist ausgehend von der Schneide 38 mit einer sacklochartigen, sich parallel zur Längsachse 16 erstreckenden Ausnehmung versehen, die als Gewebespeicher 52 für mit der Knochenstanze 10 entferntes Gewebe dient. Mit der Schneide ausgestanztes Gewebe 54 wird durch eine distalseitige Öffnung 56 des Gewebespeichers 52 in diesen hineingeschoben und bei weiterem Ausstanzen von Gewebe 54 in proximaler Richtung vorgeschoben, das heißt in Richtung des Pfeils A in Figur 1. Bei dem in den Figuren dargestellten Ausführungsbeispiel ist zum Entleeren des Gewebespeichers 52 eine Entleerungsöffnung 58 vorgesehen, welche durch eine schräg zur Längsachse 16 verlaufende Bohrung der Stanze 16 gebildet wird, welche eine Fluidverbindung zwischen dem Gewebespeicher 52 und einer Umgebung des Stanzwerkzeugs 14 herstellt. Ausgestanztes und im Gewebespeicher 52 gespeichertes Gewebe 54 kann durch die Entleerungsöffnung 58 nach außen abgegeben werden. Bei einer alternativen, nicht dargestellten Ausführungsform der Erfindung kann in der Stanze 36 ein Auswerfer in Form eines parallel zur Längsachse 16 verschieblichen Stabes vorgesehen sein, mit welchem ausgehend vom proximalen Ende des Gewebespeichers 52 in diesem gespeichertes Gewebe 54 in distaler Richtung durch die Öffnung 56 herausgeschoben werden kann.

Der Griffteil 12 der Knochenstanze 10 ist im wesentlichen in Form eines einem Pistolengriff ähnlichen Gehäuses ausgebildet. Er umfaßt einen sich parallel zur Längsachse 16 erstreckenden, langgestreckten quaderförmigen oberen Gehäuseteil 60, welcher eine in distaler Richtung weisende Öffnung 66 aufweist und einen im wesentlichen langgestreckten, quaderförmigen Aufnahmeraum 64 für das Kupplungsstück 22 des Schafts 18 bildet. Quer dazu und leicht in proximaler Richtung geneigt steht vom oberen Gehäuseteil 60 ein Griff 62 ab.

Der obere Gehäuseteil 60 umfaßt einen Schiebedeckel 70, welcher parallel zur Längsachse 16 mittels zweier in Form rippenartiger Vorsprünge ausgebildeter Führungen beidseitig in Längsnuten an sich parallel zur Längsachse 16 erstreckenden Seitenwänden 72 des oberen Gehäuseteils 60 geführt ist. In seiner distalsten Stellung bedeckt der Schiebedeckel 70 den Aufnahmeraum 64 vollständig, in seiner proximalsten Stellung gibt er den Aufnahmeraum 64 überwiegend frei, so daß eine Einlegeöffnung gebildet wird, durch die das Kupplungsstück 22 in einer Richtung quer zur Längsachse 16 in den Aufnahmeraum 64 eingelegt werden kann.

Auf Innenseiten der Seitenwände 72 stehen einander gegenüberliegend zwei aufeinander zu weisende, ein zweites Kupplungselement bildende Kupplungsvorsprünge 74 ab. Sie sind im wesentlichen in Form flacher Quader ausgebildet, deren Abmessungen so gewählt sind, daß die beiden Kupplungsvorsprünge 74 zwischen die Seitenwände 30 und 32 in die Vertiefung 26 eintauchen können und im wesentlichen am Vierkant 28 anliegen. Dadurch wird das Stanzwerkzeug 14 axial am Griffteil 12 festgelegt.

Distalseitig der Kupplungsvorsprünge 74 ist der obere Gehäuseteil 60 auf seiner Unterseite mit einer nach unten geöffneten topfförmigen Vertiefung 68 versehen, die zum Aufnahmeraum 64 hin durch eine Bohrung 76 verbunden ist. Die Vertiefung 68 ist vom Aufnahmeraum 64 weg weisend von außen her mit einer Scheibe 78 versehen, welche einen radial nach innen vorstehenden Flansch bildet. Die Vertiefung 68 dient zur Aufnahme eines Sicherungsknopfes 80, welcher einen die Bohrung 76 durchsetzenden zylindrischen Bolzen 82 umfaßt, der einen Kopf 84 trägt, wobei im Übergangsbereich zwischen dem Kopf 84 und dem Bolzen 82 ein radial nach außen stehender Ringflansch 86 ausgebildet ist. Benachbart der Bohrung 76 stützt sich in der Vertiefung, den Bolzen 82 umgebend, eine Schraubenfeder 88 ab, welche mit Ihrem anderen Ende an der Unterseite des Kopfes 84 anliegt. Dadurch wird der Ringflansch 86 in einer Grund- oder Ruhestellung gegen die Scheibe 78 gedrückt. Gegen die Wirkung der Schraubenfeder 88 kann der Sicherungsknopf 80 in Richtung auf den Aufnahmeraum 64 hin bewegt werden. Der Sicherungsknopf 80 ist zudem derart angeordnet, daß er bei in den Aufnahmeraum 64 eingelegtem Kupplungsstück 22 in der Grundstellung, in welcher die Schraubenfeder 88 den Ringflansch 86 gegen die Scheibe 78 drückt, die Sicherungsbohrung 34 im wesentlichen formschlüssig ausfüllt. Der Sicherungsknopf 80 kann bei eingelegtem Kupplungsstück 22 jedoch nur in Richtung auf den Aufnahmeraum 64 hin bewegt werden, wenn eine den zylindrischen Teil des die Durchgangsbohrung 24 durchsetzenden Stanzwerkzeugs 14 in Umfangsrichtung umgebende Ringnut 90 mit den Sicherungsbohrungen 34 in radialer Richtung überlappt. Dies ist, wie in Figur 1 dargestellt, dann der Fall, wenn das Stanzwerkzeug 14 relativ zum Schaft 18 seine proximalste Stellung einnimmt, das heißt, wenn der Abstand zwischen der Schneide 38 und der Schneidplatte 20 maximal ist. Wird der Sicherungsknopf 80 dann gedrückt, so taucht ein vorderes Ende des Bolzens 82 in die Ringnut 90 ein, wodurch eine Bewegung des Stanzwerkzeugs 14 parallel zur Längsachse 16 verhindert wird.

Eine insgesamt mit dem Bezugszeichen 92 versehene pneumatische Antriebsvorrichtung ist im wesentlichen im beziehungsweise am Griff 62 angeordnet. Die Antriebsvorrichtung 92 umfaßt einen auf einer in distaler Richtung weisenden Seite des Griffs 62, direkt unterhalb des oberen Gehäuseteils 60 angeordneten und um eine quer zur Längsachse 16 schwenkbar gelagerten Betätigungshebel 94, ein mit dem Betätigungshebel 94 betätigbares Schaltventil 96 sowie eine Antriebseinheit, welche einen ersten Pneumatikzylinder 98 und eine zweiten Pneumatikzylinder 100 umfaßt.

Die beiden Pneumatikzylinder 98 und 100 sind jeweils doppeltwirkend ausgebildet, wobei der erste Pneumatikzylinder 98 einen zylindrischen Kolbenraum 102 umfaßt, in welchem ein mittels zwei Dichtringen 104 abgedichteter Kolben 106 parallel zur Symmetrieachse des Kolbenraums 102 verschiebbar ist. Der Kolbenraum 102 ist in gleicher Weise wie der Griff 62 etwas gegenüber der Längsachse 16 geneigt. Vom Kolben 106 steht eine Kolbenstange 106 in Richtung auf den Aufnahmeraum 64 hin weisend ab, welche in einer Kolbenbohrung 110 geführt ist, die den Kolbenraum 102 mit dem Aufnahmeraum 64 verbindet. Ein Kolbenraum 112 schließt sich direkt an den Kolbenraum 102 an, und zwar mit einem etwas größeren Innendurchmesser. In diesem ist ein verschieblich gelagerter Kolben 114 mittels zweier Dichtringe 116 abgedichtet und über eine Kolbenstange 118 direkt mit dem Kolben 106 verbunden. Die beiden Pneumatikzylinder 98 und 100 bilden so eine insgesamt rotationssymmetrische Kolben-Zylinder-Einheit. Die Kolbenräume 102 und 112 sind durch eine Dichtscheibe 120 getrennt, die von der Kolbenstange 118 durchsetzt wird.

An die Kolbenstange 108 schließt sich in Richtung auf den Aufnahmeraum 64 hin weisend eine quaderförmige Verlängerung 122 an, welche einen quer zur Längsachse 16 und zur Längsachse der Kolbenstange 108 beidseitig vorstehenden Antriebsstift 124 trägt. Parallel zum Antriebsstift 124 verlaufend ist im Übergangsbereich des oberen Gehäuseteils 60 zum Griff 62 eine Lagerwelle 126 angeordnet, die zur Lagerung eines L-förmigen Winkelhebels 128 dient. Ein erster Hebelarm 130 des Winkelhebels 128 weist im wesentlichen in Richtung auf den Aufnahmeraum 64 hin, ein zweiter Hebelarm 132 ist im wesentlichen parallel zum Aufnahmeraum 64 in proximaler Richtung ausgerichtet.

Freie Enden der beiden Hebelarme 130 und 132 sind jeweils in Richtung auf die Lagerwelle 126 parallel zu einer Symmetrieebene der Knochenstanze 10 geschlitzt, so daß die freien Enden jeweils U-förmig ausgebildet sind. Der Winkelhebel 128 ist derart angeordnet, daß das geschlitzte freie Ende des ersten Hebelarms 130, welches in Form zweier scheibenförmiger Mitnehmer 134 ausgebildet ist, den Kupplungsvierkant 44 zwischen dem Flansch 42 und der Abschlußplatte 46 beidseitig umgreift, seitlich aber nicht über den Flansch 42 und die Abschlußplatte 46 vorsteht. Der erste Hebelarm 130 ist zudem derart gegenüber den Mitnehmern 134 verjüngt, daß nur diese in Anlage mit dem Flansch 42 und der Abschlußplatte 46 kommen können. Das geschlitzte Ende des zweiten Hebelarms 132 ist mit jeweils einem langlochartigen Schlitz 136 versehen und umgibt die Verlängerung 122, wobei der Antriebsstift 124 in die Schlitze 136 eintaucht und in diesem geführt ist.

Der zweite Hebelarm 132 ist etwa doppelt so lang wie der erste Hebelarm 130. Somit bildet der Winkelhebel 128 einerseits eine Kraftumlenkeinheit einer Antriebskraft, welche in Richtung des Pfeils B in Figur 1 von dem Pneumatikzylinder 98 und 100 erzeugt werden kann, in eine parallel zur Längsachse 16 verlaufende Antriebsrichtung, die durch den Pfeil C in Figur 1 symbolisiert wird. Gleichzeitig bildet der Winkelhebel 128 auch eine Übersetzungseinheit, mit welcher die von der Kolben-Zylinder-Einheit erzeugte Antriebskraft aufgrund des Längenverhältnisses der Hebelarme 130 und 132 verdoppelt wird.

Zur Steuerung der Pneumatikzylinder 98 und 100 dient das Schaltventil 96, welches im Bereich eines vom Aufnahmeraum 64 weg weisenden Endes des Griffs 62 parallel zum zweiten Pneumatikzylinder 100 angeordnet ist und einen vom Betätigungselement 94 betätigbaren, parallel zu den Kolben 106 und 114 verschiebbaren Stößel 138 umfaßt. Das Schaltventil 96 ist insgesamt rotationssymmetrisch ausgebildet, wobei der Stößel 138 das Schaltventil 96 durchsetzend, langgestreckt zylindrisch ausgebildet ist und eine Mehrzahl von Ringnuten definierter Tiefe trägt. Ein vom Betätigungselement 94 weg weisendes Ende des Stößels 138 ist mit einem Sackloch 140 versehen, in welchem sich eine Schraubenfeder 142 abstützt. Der Stößel 138 kann entgegen der Wirkung der Schraubenfeder 142 mittels des Betätigungshebels 94 in einer Richtung vom Aufnahmeraum 94 weg verschoben werden, bis er an eine Scheibe 144 anschlägt, welche durch eine zweite Schraubenfeder 146 gegen eine vom Betätigungselement 94 weg weisende Ringwand eines zylindrischen Ventilraums 148 gedrückt wird. Sowohl die Schraubenfeder 142 als die Schraubenfeder 146 stützen sich, eine Einlaßöffnung 150 des Ventilraums 148 umgebend am Boden desselben ab, welcher in Richtung auf das Betätigungselement 94 hin weist und der Ringwand gegenüberliegt. Die Schraubenfeder 146 weist eine Federkonstante auf, die die der Schraubenfeder 142 um ein mehrfaches übersteigt. So kann bei Betätigung des Betätigungshebels 94 von einer Bedienperson aufgrund der durch die Schraubenfedern 142 und 146 erzeugten Rückstellkräfte auf den Stößel 138 auf eine Schaltstellung des Schaltventils 96 geschlossen werden. Ausgehend von einer unbetätigten Stellung des Betätigungshebels 94 wird der Stößel zunächst nur entgegen der Schraubenfeder 192 gedrückt, bis der Stößel 138 an der Scheibe 144 anschlägt. Wird der Betätigungshebel 94 weiter verschwenkt, so nimmt der Stößel 138 die Scheibe 144 mit und es wird zusätzlich die Schraubenfeder 146 komprimiert. Da deren Federkonstante um ein vielfaches größer ist als die der Schraubenfeder 142, erhält eine Bedienperson somit eine taktile Rückmeldung, daß nun in eine zweite Schaltstellung gewechselt wurde.

Die Einlaßöffnung 150 des Schaltventils 96 ist in nicht dargestellter Weise mit einer Druckluftzufuhrleitung 152 verbunden, welche den Griffteil 12 mit einer nicht dargestellten Druckluftquelle verbindet. Nicht näher bezeichnete Steuereinlässe und Steuerauslässe des Schaltventils 96 sind mit den beiden Pneumatikzylindern 98 und 100 verbunden, um die nachfolgende Funktionsweise der Knochenstanze zu realisieren.

In einer unbetätigten Stellung des Betätigungshebels 94 wird der erste Pneumatikzylinder 98 derart mit Druckluft beaufschlagt, daß der Kolben 106 gegen die Dichtscheibe 120 bewegt wird. Die Knochenstanze nimmt dann ihre geöffnete Stellung ein, das heißt der Mitnehmer 134 hält die Abschlußplatte 46 der Stanze 36 in ihrer proximalsten Stellung. Der Abstand zwischen der Schneide 38 und der Schneidplatte 20 ist maximal.

Wird der Betätigungshebel 94 entgegen der Schraubenfeder 142 bewegt, ohne daß der Stößel 138 an der Scheibe 144 anschlägt, wird nur der Kolben 106 in Richtung des Pfeils B in Figur 1 mittels Druckluftbeaufschlagung zwischen dem Kolben 106 und der Drehscheibe 120 bewegt. Ein Verhältnis der Wirkungsquerschnitte des ersten Pneumatikzylinders 98 und des zweiten Pneumatikzylinders 100 beträgt etwa 1 : 4, so daß zunächst nur eine Antriebskraft in Höhe von etwa 20 Prozent einer maximal möglichen Antriebskraft erzeugt und über den Winkelhebel 128 auf die Stanze 36 übertragen wird. Die so verminderte, auf die Stanze 36 wirkende Kraft reicht typischerweise nicht dazu aus, um zu entfernendes Gewebe 54, beispielsweise Knochenteile, zu durchtrennen. Die Kraft reicht, wie im gestrichelt umgrenzten Bereich D in Figur 1 dargestellt, nur dazu aus, die Schneide 38 an das Gewebe heranzuführen. In dieser ersten Betätigungsstellung des Betätigungshebels kann eine Bedienperson der Kinochenstanze 10 damit die Schneidplatte 20 in gewünschter Weise an das zu entfernende Gewebe 54 anlegen, ohne daß es bereits zu einer Durchtrennung des Gewebes 54 kommt.

Ist die Schnittposition bestimmt, dann kann der Betätigungshebel 94 voll durchgedrückt werden. Der Stößel 138 wird nunmehr auch entgegen der Schraubenfeder 146 bewegt, was die Bedienperson durch die größere aufzubringende Betätigungskraft spürt, die erforderlich ist, um den Betätigungshebel 94 zu verschwenken. Das Schaltventil 96 nimmt dann eine Schaltstellung ein, in welcher sowohl der erste Pneumatikzylinder 98 als auch der zweite Pneumatikzylinder 100 mit Druckluft beaufschlagt werden, und zwar derart, daß beide Kolben 106 und 114 in Richtung des Pfeils B bewegt werden. Damit wird eine Maximalkraft der Antriebsvorrichtung 92 über den Winkelhebel 128 auf die Stanze 36 übertragen, welche geeignet ist, Gewebe 54 in gewünschter Weise zu durchtrennen.

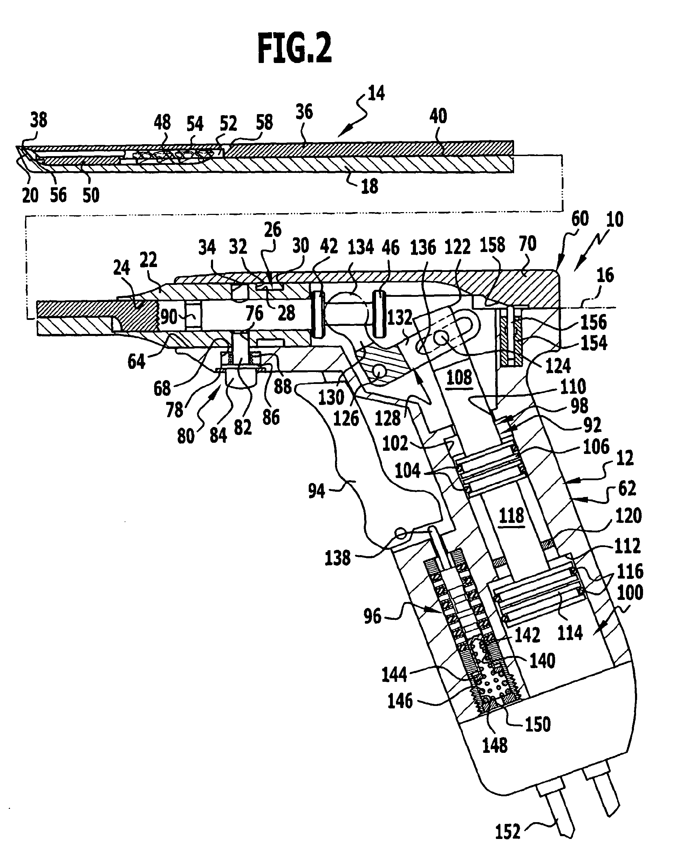

Wird der Betätigungshebel 94 wieder entlastet, so drücken die Schraubenfedern 142 und 146 den Stößel 138 in seine Ausgangsstellung zurück, die Stanze 36 wird dann wieder durch Druckbeaufschlagung des Pneumatikzylinders 98 in einer zum Pfeil B entgegengesetzten Richtung beaufschlagt, wodurch die Stanze 36 von ihrer in Figur 2 dargestellten Schneidstellung über ihre im Bereich D in Figur 1 dargestellte Zwischenstellung in ihre in Figur 1 dargestellte Grundstellung, also ihre proximalste Stellung, zurücküberführt wird.

Um ein versehentliches Betätigen der Antriebsvorrichtung 92 durch Drücken des Betätigungshebels 94 durch eine Bedienperson auszuschließen, ist im proximalen Bereich des oberen Gehäuseteils 60 ein Sicherheitsventil 154 angeordnet. Das Sicherheitsventil 154 umfaßt einen quer zur Längsachse 16 verschiebbar gelagerten, gegen eine Innenseite des Deckels 70 federnd vorgespannten Ventilstößel 156. Ist der Deckel, wie in Figur 1 dargestellt, geschlossen, nimmt der Ventilstößel 156 eine Stellung ein, bei welcher er eine Fluidverbindung zwischen der Druckluftzufuhrleitung 152 und der Einlaßöffnung 150 des Schaltventils 96 herstellt. Wird der Deckel 70 jedoch geöffnet, gleitet das aus dem Sicherheitsventil 154 vorstehende Ende des Ventilstößels 156 an einer Anlaufschräge 158 auf der Innenseite des Deckels 70 entlang, wodurch der federnd vorgespannte Ventilstößel 156 weiter aus einem Körper des Sicherheitsventils 154 herausbewegt wird. Dadurch wechselt das Sicherheitsventil 154 seine Schaltstellung, es unterbricht nunmehr eine Verbindung zwischen der Druckluftzufuhrleitung 152 und der Einlaßöffnung 150 des Schaltventils 96, so daß eine Betätigung des Betätigungshebels 94 wirkungslos bleibt, da die Antriebsvorrichtung 92 somit von der Druckluftquelle getrennt ist. Erst dann, wenn der Schiebedeckel 70 wieder vollständig geschlossen ist, wird der Ventilstößel 156 so weit in den Körper des Sicherheitsventils 154 hineingedrückt, daß das Sicherheitsventil 154 wieder die Schaltstellung einnimmt, in welcher die Druckluftzufuhrleitung 152 in Fluidverbindung mit der Einlaßöffnung 150 des Schaltventils 96 steht.

Muß die Knochenstanze 10 während eines Einsatzes gereinigt werden, beispielsweise weil sich Gewebe 54 zwischen der Schneide 38 und der Schneidplatte 20 verklemmt hat, muß ein Operateur die Knochenstanze 10 nicht aus der Hand legen. Eine dem Operateur assistierende Person kann einen Zwischenraum zwischen der Schneide 38 und der Schneidplatte 20 reinigen, ohne Gefahr zu laufen, verletzt zu werden, selbst dann, wenn der Operateur den Betätigungshebel 94 betätigt. In einer unbetätigten Stellung des Betätigungshebels 94 überlappen die Ringnut 90 und die Sicherungsbohrungen 34. Die dem Operateur assistierende Person kann nun den Sicherungsknopf 80 entgegen der Wirkung der Schraubenfeder 88 drücken, so daß der Bolzen 82 in die Ringnut 90 eintaucht. Eine Bewegung der Stanze 36 in distaler Richtung wird dadurch verhindert, selbst dann, wenn der Operateur den Betätigungshebel 94 drücken würde. Ist das zu entfernende Gewebeteil 54 abgeschnitten entfernt, kann die dem Operateur assistierende Person den Sicherungsknopf 80 wieder loslassen und der Operateur kann den chirurgischen Eingriff fortsetzen. Der beschriebene Reinigungsvorgang läßt sich besonders leicht durchführen, da der Sicherungsknopf 80 weit genug entfernt vom Betätigunshebel 94 angeordnet ist, insbesondere distalseitig desselben, so daß der Sicherungsknopf 80 für die dem Operateur assistierende Person stets frei zugänglich ist.

Durch die besondere Ausgestaltung des Kupplungsstücks 22, insbesondere durch Vorsehen des Vierkants 28, ist es möglich, das Stanzwerkzeug 14 in vier unterschiedlichen Stellungen mit dem Griffteil 12 zu verbinden. In den Figuren 1 bis 5 ist das Stanzwerkzeug 14 mit dem Griffteil 12 derart verbunden, daß ein Zwischenraum zwischen der Schneidplatte 20 und der Schneide 38 nach oben weist. Die beiden Teile der Knochenstanze 10 können jedoch auch so miteinander verbunden werden, daß der Zwischenraum nach unten oder nach einer der beiden Seiten der Knochenstanze 10 weist. Zum Ändern einer Relativposition des Stanzwerkzeugs 14 zum Griffteil 12 muß lediglich der Schiebedeckel 70 in proximaler Richtung verschoben werden, bis der Aufnahmeraum 63 geöffnet und das Stanzwerkzeug 14 entnommen werden kann. Das Stanzwerkzeug kann dann um 90°, 180° oder 270° gedreht und wieder in den Aufnahmeraum 64 eingesetzt werden. Verschieben des Deckels 70 in distaler Richtung verschließt den Aufnahmeraum 64 und sichert zudem das Kupplungsstück 22 am Griffteil 12.

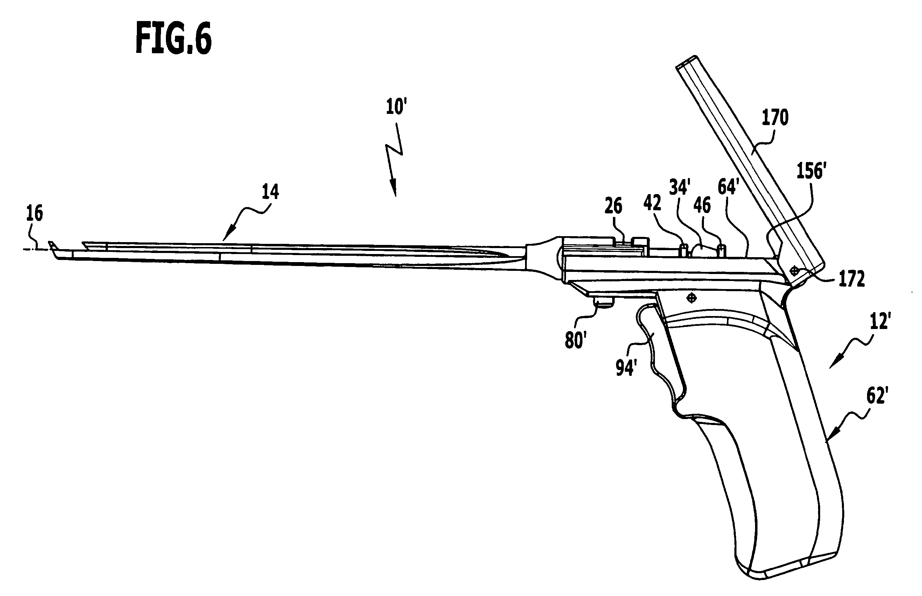

In Figur 6 ist eine insgesamt mit dem Bezugszeichen 10' versehene zweite Ausführungsform einer erfindungsgemäßen Knochenstenze dargestellt, bei welcher das Stanzwerkzeug 14 identisch mit dem Stanzwerkzeug 14 der Knochenstanze 10 ausgebildet ist, sich jedoch das Griffteil 12' vom Griffteil 12 der Knochenstanze 10 etwas unterscheidet. Teile die sowohl beim Griffteil 12 als auch beim Griffteil 12' identisch ausgebildet sind, sind beim Griffteil 12' mit denselben Bezugszeichen, jedoch mit einem hochgestellten Strich (') versehen.

Einziger und wesentlicher Unterschied des Griffteils 12' gegenüber dem Griffteil 12 ist der Klappdeckel 170. Er ist um eine am proximalen Ende des Aufnahmeraums 64' um eine durch zwei vom Deckel 170 senkrecht abstehende und aufeinander zu weisende, in korrespondierenden Bohrungen der Seitenwände 72 gehaltene Zapfen definierte Schwenkachse 172 verschwenkbar gelagert. In Figur 6 ist der Klappdeckel 170 in einer geöffneten Stellung dargestellt, in welcher er den Aufnahmeraum 64' freigibt, um das Stanzwerkzeug 14 zu entnehmen. Mittels einer nicht näher dargestellten Verriegelung kann der Klappdeckel 170 in seiner den Aufnahmeraum 64' bedeckenden und verschließenden Stellung gesichert werden.

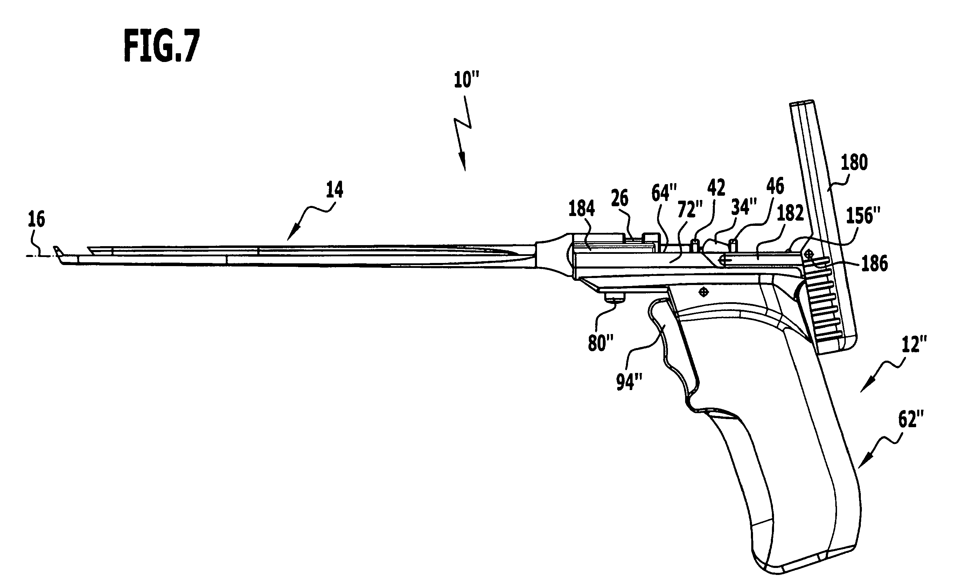

Ein drittes Ausführungsbeispiel einer erfindungsgemäßen Knochenstanze ist in Figur 7 insgesamt mit dem Bezugszeichen 10" versehen. Die Knochenstanze 10" umfaßt ebenfalls ein mit dem Stanzwerkzeug 14 der Knochenstanze 10 identisches Stanzwerkzeug 14 sowie ein insgesamt mit dem Bezugszeichen 12" versehenes Griffteil, das sich vom Griffteil 12 der Knochenstanze 10 durch die Ausgestaltung des Deckels 180 unterscheidet. Identische Teile am Griffteil 12 sowie am Griffteil 12" sind daher wiederum mit identischen Bezugszeichen mit zusätzlich zwei hochgestellten Strichen (") versehen.

Am distalen Ende der Seitenwände 72" sind symmetrisch zwei Führungsrippen 184 auf beiden Seiten des Aufnahmeraums 64" angeordnet, welche eine Längsführung parallel zur Längsachse 16 für ein distales Ende des Deckels 180 bilden. Zu diesem Zweck sind am Deckel 180 zu den beiden Führungsrippen 184 korrespondierende, nicht näher dargestellte Längsvorsprünge angeordnet. Im proximalen Endbereich der Seitenwände 72" sind diese mit seitlich geöffneten, sich parallel zu den Führungsrippen 184 erstreckenden Führungsnuten 182 versehen, in die jeweils mittig und aufeinander zu weisend, am Deckel 180 abstehende Führungszapfen eintauchen und somit ebenfalls eine Führung des Deckels 118 parallel zur Längsachse 16 bilden.

Ausgehend von einer nicht dargestellten, den Aufnahmeraum 64" schließenden Stellung des Deckels 180 kann dieser zunächst nur in proximaler Richtung verschoben werden, und zwar über eine Strecke, die der Länge der Führungsrippen 184 am distalen Ende der Seitenwände 72" entspricht. Sobald der Deckel 180 außer Eingriff mit den Führungsrippen 184 steht, kann dieser um eine von den Führungszapfen definierte Schwenkachse 186 verschwenkt und nach hinten weggeklappt werden, um den Aufnahmeraum 64" ganz freizugeben. Das Stanzwerkzeug 14 kann jetzt gegen ein anderes ausgetauscht werden. Ist das andere Stanzwerkzeug 14 in den Aufnahmeraum 64" eingesetzt, wird zum Verschließen des Aufnahemraums 64" der Deckel 180 zunächst um die Schwenkachse 186 verschwenkt und dann parallel zur Längsachse 16 in distaler Richtung verschoben, wobei darauf zu achten ist, daß das distale Ende des Deckels 180 wieder in die Führungsrippen 184 eingreift. Diese bilden somit gleichzeitig eine Führung und eine Verriegelung für den Deckel 180 an den Seitenwänden 72'.

Im übrigen sind die Griffteile 12' und 12" der Knochenstanze 10' und 10" identisch mit dem Griffteil 12 der Knochenstanze 10 ausgebildet.

## Patentansprüche

1. Chirurgisches Instrument (10) zum Entfernen von Knochen, Knorpel oder derartigem Gewebe (54), mit einem sich in einer Längsrichtung (16) erstreckenden Schaft (18), welcher an seinem distalen Ende eine quer zur Längsrichtung oder relativ zu dieser geneigte Schneidplatte (20) trägt, mit einem am Schaft (18) verschiebbar gelagerten Schneidelement (36), welches an seinem distalen Ende eine in Richtung auf die Schneidplatte (20) weisende, zum Schneiden von Gewebe (54) an die Schneidplatte (20) heranführbare Schneide (38) trägt, wobei am proximalen Ende des Schafts (18) ein erstes Kupplungselement (28) angeordnet ist zum lösbaren, formschlüssigen Verbinden mit einer Antriebsvorrichtung (12), welche ein mit dem ersten Kupplungselement zusammenwirkendes zweites Kupplungselement (74) aufweist, **dadurch gekennzeichnet, dass** das erste Kupplungselement (28) ein am proximalen Ende des Schaftes angeordnete, sich in Umfangsrichtung erstreckende, einen Nutboden (28) sowie in distaler und proximaler Richtung weisenden Nutseitenwanden (30, 32) aufweisende Vielkantige Nut ist, dessen Außenflächen von einer Längsachse (16) des Schafts (18) radial nach außen weisen, wobei die Nutseitenwanden als in Längsrichtung (16) des Schafts (18) wirkende Anschläge (30, 32) dienen.

2. Chirurgisches Instrument nach Anspruch 1, **dadurch gekennzeichnet, dass** der Vielkant ein (28) Vierkant, ein Sechskant oder ein Achtkant ist.

3. Chirurgisches Instrument nach einem der vorangegangenen Ansprüche, **dadurch gekennzeichnet, dass** das proximale Ende des Schafts (18) eine zur Längsrichtung (16) parallele Symmetrieachse aufweist.

4. Chirurgisches Instrument nach einem der vorangegangenen Ansprüche, **dadurch gekennzeichnet, dass** der Schaft (18) an seinem proximalen Ende hülsenartig geformt ist und dass das Schneidelement (36) das hülsenartige Ende (22) des Schafts (18) durchsetzt.

5. Chirurgisches Instrument nach Anspruch 4, **dadurch gekennzeichnet, dass** der in distaler Richtung wirkender Anschlag (30) proximalseitig des ersten Kupplungselements (28) ange-ordnet ist und dass der in proximaler Richtung wirkender Anschlag (32) distalseitig des ersten Kupplungselements (28) angeordnet ist.

6. Chirurgisches Instrument nach einem der vorangegangenen Ansprüche, **dadurch gekennzeichnet, dass** das proximale Ende (22) des Schafts (18) mindestens eine sich in radialer Richtung erstreckende Durchbrechung (34) aufweist, dass das Schneidelement (36) mindestens eine in radialer Richtung weisende Vertiefung (90) aufweist und dass in einer geöffneten Stellung des Instruments (10), in welcher die Schneide (38) von der Schneidplatte (20) beabstandet ist, die mindestens eine Durchbrechung (34) und die Vertiefung (90) einander mindestens teilweise überlappen.

7. Chirurgisches Instrument nach Anspruch 9, **dadurch gekennzeichnet, dass** die Durchbrechung (34) eine Bohrung und die Vertiefung (90) eine Sacklochbohrung, ein sacklochartiges Langloch oder eine Ringnut sind.

8. Chirurgisches Instrument nach Anspruch 10, **dadurch gekennzeichnet, dass** ein Durchmesser der Bohrung (34) und eine Breite der Ringnut (90) oder ein Durchmesser der Sacklochbohrung übereinstimmen oder im Wesentlichen übereinstimmen und dass die Bohrung (34) und die Ringnut (90) oder die Sacklochbohrung in der geöffneten Stellung in Deckung bringbar sind.

9. Chirurgisches Instrument nach einem der Ansprüche 9 bis 11, **dadurch gekennzeichnet, dass** die Durchbrechung (34) benachbart dem ersten Kupplungselement (28) angeordnet ist.

10. Chirurgisches Instrument nach einem der Ansprüche 9 bis 12, **dadurch gekennzeichnet, dass** die Durchbrechung (34) distalseitig des ersten Kupplungselements (28) angeordnet ist.

11. Chirurgisches Instrument nach einem der vorangegangenen Ansprüche, **dadurch gekennzeichnet, dass** das Schneidelement (36) an seinem proximalen Ende ein drittes, mit einem Antriebselement (98, 100) der Antriebsvorrichtung (12) lösbar verbindbares Kupplungselement (44) trägt und dass sich an das dritte Kupplungselement (44) in Längsrichtung (16) des Schafts (18) wirkende Anschläge (42, 46) anschließen.

12. Chirurgisches Instrument nach Anspruch 14, **dadurch gekennzeichnet, dass** ein in distaler Richtung wirkender Anschlag (46) proximalseitig des dritten Kupplungselements (44) angeordnet ist und dass ein in proximaler Richtung wirkender Anschlag (42) distalseitig des ersten Kupplungselements (44) angeordnet ist.

13. Chirurgisches Instrument nach Anspruch 14, **dadurch gekennzeichnet, dass** ein in distaler Richtung wirkender Anschlag distalseitig des dritten Kupplungselements angeordnet ist und dass ein in proximaler Richtung wirkender Anschlag proximalseitig des dritten Kupplungselements angeordnet ist.

14. Chirurgisches Instrument nach einem der Ansprüche 14 bis 16, **dadurch gekennzeichnet, dass** das dritte Kupplungselement (44) ein Vielkant ist.

15. Chirurgisches Instrument nach einem der vorangegangenen Ansprüche, **dadurch gekennzeichnet, dass** die Schneide (38) eine in sich geschlossene ringförmige Schneide (38) ist.

16. Chirurgisches Instrument nach einem der vorangegangenen Ansprüche, **dadurch gekennzeichnet, dass** das Schneidelement (36) eine sich ausgehend von seinem distalen Ende in proximaler Richtung erstreckende Schneidgutausnehmung (52) zum Aufnehmen von entferntem Gewebe (54) aufweist und dass die Schneide (38) eine Öffnung (56) der Schneidgutausnehmung (52) mindestens teilweise umgibt.

17. Chirurgisches Instrument nach Anspruch 19, **dadurch gekennzeichnet, dass** die Schneidgutausnehmung (52) sacklochartig ausgebildet ist und dass ein in Längsrichtung (16) am Schneidelement (36) verschiebbarer Auswerfer vorgesehen ist, welcher von einer zurückgezogenen Stellung, in welcher er die Schneidgutausnehmung (52) freigibt, in eine Auswurfstellung, in welcher er die Schneidgutausnehmung (52) vollständig oder im wesentlichen vollständig ausfüllt, verschiebbar ist und umgekehrt.

18. Chirurgisches Instrument nach einem der Ansprüche 19 oder 20, **dadurch gekennzeichnet, dass** ein proximales Ende der Schneidgutausnehmung (52) mit einer seitlichen Auswurföffnung (58) versehen ist.

19. Chirurgisches Instrument nach einem der Ansprüche 19 bis 21, **dadurch gekennzeichnet, dass** das Schneidelement (36) im Bereich der Schneidgutausnehmung (52) seitlich mit mindestens einer Sichtöffnung versehen ist.

20. Chirurgisches Instrument nach Anspruch 22, **dadurch gekennzeichnet, dass** die mindestens eine Sichtöffnung eine Breite quer zur Längsrichtung (16) aufweist, die kleiner als ein Innendurchmesser der Schneidgutausnehmung (52) ist.

21. Chirurgisches Instrument nach einem der Ansprüche 22 oder 23, **dadurch gekennzeichnet, dass** mehrere langlochartige Sichtöffnungen vorgesehen sind.

22. Chirurgisches Instrument nach einem der Ansprüche 22 bis 24, **dadurch gekennzeichnet, dass** die mindestens eine Sichtöffnung distalseitig benachbart dem proximalen Ende der Schneidgutausnehmung (52) angeordnet ist.

23. Chirurgisches Instrument nach einem der vorangegangenen Ansprüche, **dadurch gekennzeichnet, dass** eine Führung (24, 48) vorgesehen ist zum Führen einer Bewegung des Schneidelements (36) relativ zum Schaft (18) in Längsrichtung (16).

24. Chirurgisches Instrument nach einem der vorangegangenen Ansprüche, **dadurch gekennzeichnet, dass** die Schneide (38) und/oder die Schneidplatte (20) mit einer abriebfesten Beschichtung versehen sind.

25. Chirurgisches Instrument nach Anspruch 27, **dadurch gekennzeichnet, dass** die abriebfeste Beschichtung Titannitrid (TiN) ist oder Titannitrid (TiN) enthält.

26. Chirurgisches Instrument nach einem der vorangegangenen Ansprüche, **dadurch gekennzeichnet, dass** am Schaft (18) eine mit einer Dekodiereinheit der Antriebsvorrichtung (92) zusammenwirkende Kodiereinheit vorgesehen ist zur Kodierung der Art oder des Typs des Schneidelements (36).

27. Chirurgisches Instrument nach Anspruch 29, **dadurch gekennzeichnet, dass** die Kodiereinheit mindestens einen vom Schaft (18) abstehenden Vorsprung umfasst.

## Claims

1. Surgical instrument (10) for removing bone, cartilage or similar tissue (54) with a shaft (18) that extends in a longitudinal direction (16) and at its distal end carries a cutting plate (20) inclined transversely to the longitudinal direction or relative thereto, with a cutting element (36) which is movably mounted on the shaft (18) and carries at its distal end a blade (38) pointing in the direction of the cutting plate (20) and movable toward the cutting plate (20) in order to cut tissue (54), a first coupling element (28) being arranged at the proximal end of the shaft (18) for detachable positive connection to a driving device (12) which has a second coupling element (74) interacting with the first coupling element, **characterized in that** the first coupling element (28) is a polyhedral groove which is arranged at the proximal end of the shaft, extends in the circumferential direction and has a groove base (29) and also groove sidewalls (30, 32) pointing in the distal and proximal direction, the outer surfaces of which groove point radially outward from a longitudinal axis (16) of the shaft (18), the groove sidewalls serving as stops (30, 32) acting in the longitudinal direction (16) of the shaft (18).

2. Surgical instrument according to claim 1, **characterized in that** the polyhedron (28) is a square, a hexagon or an octagon.

3. Surgical instrument according to any one of the preceding claims, **characterized in that** the proximal end of the shaft (18) has an axis of symmetry parallel to the longitudinal direction (16).

4. Surgical instrument according to any one of the preceding claims, **characterized in that** at its proximal end the shaft (18) is sleeve-like and **in that** the cutting element (36) passes through the sleeve-like end (22) of the shaft (18).

5. Surgical instrument according to claim 4, **characterized in that** the stop (30) acting in the distal direction is arranged on the proximal side of the first coupling element (28) and **in that** the stop (32) acting in the proximal direction is arranged on the distal side of the first coupling element (28).

6. Surgical instrument according to any one of the preceding claims, **characterized in that** the proximal end (22) of the shaft (18) has at least one radially extending opening (34), that the cutting element (36) has at least one radially pointing recess (90) and that in an open position of the instrument (10) in which the blade (38) is at a distance from the cutting plate (20), the at least one gap (34) and the recess (90) at least partially overlap each other.

7. Surgical instrument according to claim 6, **characterized in that** the opening (34) is a drilled hole and the recess (90) is a pocket hole boring, a pocket hole-like elongated slot or an annular groove.

8. Surgical instrument according to claim 7, **characterized in that** a diameter of the drilled hole (34) and a width of the annular groove (90) or a diameter of the pocket hole boring correspond or substantially correspond and **in that** the drilled hole (34) and the annular groove (90) or the pocket hole boring can be brought into alignment in the open position.

9. Surgical instrument according to any one of claims 6 to 8, **characterized in that** the opening (34) is arranged adjacent to the first coupling element (28).

10. Surgical instrument according to any one of claims 6 to 9, **characterized in that** the opening (34) is arranged on the distal side of the first coupling element (28).

11. Surgical instrument according to any one of the preceding claims, **characterized in that** at its proximal end the cutting element (36) supports a third coupling element (44) which can be detachably connected to a drive element (98, 100) of the driving device (12) and **in that** stops (42, 46) acting in the longitudinal direction (16) of the shaft (18) adjoin the third coupling element (44).

12. Surgical instrument according to claim 11, **characterized in that** a stop (46) acting in the distal direction is arranged on the proximal side of the third coupling element (44) and that a stop (42) acting in the proximal direction is arranged on the distal side of the first coupling element (44).

13. Surgical instrument according to claim 11, **characterized in that** a stop acting in the distal direction is arranged on the distal side of the third coupling element and that a stop acting in the proximal direction is arranged on the proximal side of the third coupling element.

14. Surgical instrument according to any one of claims of 11 to 13, **characterized in that** the third coupling element (44) is a polyhedron.

15. Surgical instrument according to any one of the preceding claims, **characterized in that** the blade (38) is a self-enclosed annular blade (38).

16. Surgical instrument according to any one of the preceding claims, **characterized in that** the cutting element (36) has a cut material recess (52), extending from its distal end in the proximal direction, for receiving removed tissue (54) and **in that** the blade (38) at least partially surrounds an opening (56) in the cut material recess (52).

17. Surgical instrument according to claim 16, **characterized in that** the cut material recess (52) is in the form of a pocket hole and that an ejector movable in the longitudinal direction (16) on the cutting element (36) is provided and can be moved from a retracted position in which it releases the cut material recess (52) to an ejection position in which it completely or substantially completely fills the cut material recess (52) and vice versa.

18. Surgical instrument according to either claim 16 or claim 17, **characterized in that** a proximal end of the cut material recess (52) is provided with a lateral ejection opening (58).

19. Surgical instrument according to any one of claims 16 to 18, **characterized in that** in the region of the cut material recess (52) the cutting element (36) is laterally provided with at least one viewing opening.

20. Surgical instrument according to claim 19, **characterized in that** the at least one viewing opening has a width transversely to the longitudinal direction (16) that is smaller than an internal diameter of the cut material recess (52).

21. Surgical instrument according to any one of claim 19 or claim 20, **characterized in that that** several elongated hole-type viewing openings are provided.

22. Surgical instrument according to any one of claims 19 to 21, **characterized in that** the at least one viewing opening is arranged distally adjacent to the proximal end of the cut material recess (52).

23. Surgical instrument according to any one of the preceding claims, **characterized in that** a guide (24, 48) is provided for guiding a movement of the cutting element (36) relative to the shaft (18) in the longitudinal direction (16).

24. Surgical instrument according to any one of the preceding claims, **characterized in that** the blade (38) and/or the cutting plate (20) are provided with an abrasion-proof coating.

25. Surgical instrument according to claim 24, **characterized in that** the abrasion-proof coating is titanium nitride (TiN) or contains titanium nitride (TiN).

26. Surgical instrument according to any one of the preceding claims, **characterized in that** a coding unit which interacts with a decoding unit of the driving device (92) is provided on the shaft (18) for coding the nature or the type of the cutting element (36).

27. Surgical instrument according to claim 26, **characterized in that** the coding unit comprises at least one projection projecting from the shaft (18).

## Revendications

1. Instrument chirurgical (10) servant à prélever des os, des cartilages ou du tissu de ce genre (54), avec une tige (18) s'étendant dans une direction longitudinale (16), laquelle porte au niveau de son extrémité distale une plaquette de coupe (20) inclinée transversalement par rapport à la direction longitudinale ou par rapport à celle-ci, avec un élément de coupe (36) logé de façon mobile au niveau de la tige (18), lequel porte, au niveau de son extrémité distale, une lame (38) pouvant être rétractée dans la direction de la plaque de coupe (20) pour couper du tissu (54) sur la plaque de coupe (20), un premier élément d'accouplement (28) étant disposé au niveau de l'extrémité proximale de la tige (18), pour un raccordement amovible à complémentarité de forme avec un dispositif de commande (12) qui comprend un second élément d'accouplement (74), coopérant avec le premier élément d'accouplement, **caractérisé en ce que** le premier élément d'accouplement (28) est une rainure polygonale disposée à l'extrémité proximale de la tige, s'étendant de façon périphérique et comprenant un fond de rainure (28) ainsi que des parois latérales de rainure dans les directions distale et proximale (30, 32), dont les surfaces extérieures s'étendent vers l'extérieur de façon radiale à partir d'un axe longitudinal (16) de la tige (18), les parois latérales de la rainure servant de butées (30,32) agissant dans la direction longitudinale (16) de la tige (18).

2. Instrument chirurgical selon la revendication 1, **caractérisé en ce que** la pièce polygonale est à (28) quatre-pans, six-pans ou huit-pans.

3. Instrument chirurgical selon l'une quelconque des revendications précédentes, **caractérisé en ce que** l'extrémité proximale de la tige (18) comprend un axe de symétrie parallèle à la direction longitudinale (16).

4. Instrument chirurgical selon l'une quelconque des revendications précédentes, **caractérisé en ce que** la tige (18) est formée au niveau de son extrémité proximale d'une sorte de douille et **en ce que** l'élément de coupe (36) traverse l'extrémité en forme de douille (22) de la tige (18).

5. Instrument chirurgical selon la revendication 4, **caractérisé en ce que** la butée (30) agissant dans une direction distale est disposée sur le côté proximal du premier élément d'accouplement (28) et **en ce que** la butée (32) agissant dans une direction proximale est disposée sur le côté distal du premier élément d'accouplement (28).

6. Instrument chirurgical selon l'une quelconque des revendications précédentes, **caractérisé en ce que** l'extrémité proximale (22) de la tige (18) comprend au moins une brèche (34) s'étendant dans une direction radiale, **en ce que** l'élément de coupe (36) comprend au moins un évidement (90) se trouvant dans une direction radiale et **en ce que** dans une position ouverte de l'instrument (10), dans laquelle la lame (38) est située à distance de la plaque de coupe (20), la au moins une brèche (34) et l'évidement (90) se chevauchent l'un l'autre au moins en partie.

7. Instrument chirurgical selon la revendication 6, **caractérisé en ce que** la brèche (34) un alésage et l'évidement (90) sont un alésage à trous borgnes, un trou oblong de type à trous borgnes ou une rainure annulaire.

8. Instrument chirurgical selon la revendication 7, **caractérisé en ce qu'**un diamètre de l'alésage (34) et une largeur de la rainure annulaire (90) ou un diamètre de l'alésage à trous borgnes correspondent ou correspondent essentiellement et **en ce que** l'alésage (34) et la rainure annulaire (90) ou l'alésage à trous borgnes dans la position ouverte peuvent être amenés en couverture.

9. Instrument chirurgical selon l'une quelconque des revendications 6 à 8, **caractérisé en ce que** la brèche (34) est disposée de façon adjacente au premier élément d'accouplement (28).

10. Instrument chirurgical selon l'une quelconque des revendications 6 à 9, **caractérisé en ce que** la brèche (34) est disposée sur le côté distal du premier élément d'accouplement (28).

11. Instrument chirurgical selon l'une quelconque des revendications précédentes, **caractérisé en ce que** l'élément de coupe (36) porte au niveau de son extrémité proximale un troisième élément d'accouplement (44) pouvant être raccordé de façon mobile à un élément de commande (98, 100) d'un dispositif de commande (12) et **en ce que** des butées (42, 46) agissant dans la direction longitudinale (16) de la tige (18) se raccordant au troisième élément d'accouplement (44).

12. Instrument chirurgical selon la revendication 11 **caractérisé en ce qu'**une butée (46) agissant dans une direction distale est disposée du côté proximal du troisième élément d'accouplement (44) et **en ce qu'**une butée (42) agissant dans une direction proximale est disposée du côté distal du premier élément d'accouplement (44).

13. Instrument chirurgical selon la revendication 11, **caractérisé en ce qu'**une butée agissant dans une direction distale est disposée du côté distal du troisième élément d'accouplement et **en ce qu'**une butée agissant dans une direction proximale est disposée du côté proximal du troisième élément d'accouplement.

14. Instrument chirurgical selon l'une quelconque des revendications 11 à 13, **caractérisé en ce que** le troisième élément d'accouplement (44) est un élément polygonal.

15. Instrument chirurgical selon l'une quelconque des revendications précédentes, **caractérisé en ce que** la lame (38) est une lame circulaire autonome (38).

16. Instrument chirurgical selon l'une quelconque des revendications précédentes, **caractérisé en ce que** l'élément de coupe (36) comprend un creux recevant une lame (52) s'étendant à partir de son extrémité distale en direction proximale (52) pour recevoir le tissu prélevé (54) et **en ce que** la lame (38) entoure au moins en partie une ouverture (56) du creux recevant une lame (52).

17. Instrument chirurgical selon la revendication 16, **caractérisé en ce que** le creux recevant une lame (52) est conçu en forme de trou borgne et **en ce qu'**un éjecteur pouvant être déplacé dans la direction longitudinale (16) au niveau de l'élément de coupe (36) est prévu et peut être déplacé et renversé à partir d'une position rétractée, dans laquelle il libère le creux recevant une lame (52), dans une position d'éjection dans laquelle il remplit complètement ou essentiellement complètement le creux recevant une lame (52).

18. Instrument chirurgical selon l'une quelconque des revendications 16 ou 17, **caractérisé en ce qu'**une extrémité proximale du creux recevant une lame (52) est prévue avec une ouverture d'éjection latérale (58).

19. Instrument chirurgical selon l'une quelconque des revendications 16 à 18, **caractérisé en ce que** l'élément de coupe (36) est prévu dans le secteur du creux recevant une lame (52), sur le côté, avec au moins une ouverture de visée.

20. Instrument chirurgical selon la revendication 19, **caractérisé en ce que** la au moins une ouverture de visée comprend une largeur transversale par rapport à la direction longitudinale (16) qui est inférieure à un diamètre intérieur du creux recevant une lame (52).

21. Instrument chirurgical selon l'une quelconque des revendications 19 ou 20, **caractérisé en ce que** plusieurs ouvertures de visée de type à trous oblongs sont prévues.

22. Instrument chirurgical selon l'une quelconque des revendications 19 à 21, **caractérisé en ce que** la au moins une ouverture de visée est disposée sur le côté distal de façon adjacente à l'extrémité proximale du creux recevant une lame (52).

23. Instrument chirurgical selon l'une quelconque des revendications précédentes, **caractérisé en ce qu'**un système de guidage (24, 48) est prévu pour guider un mouvement de l'élément de coupe (36) par rapport à la tige (18) dans une direction longitudinale (16).

24. Instrument chirurgical selon l'une quelconque des revendications précédentes, **caractérisé en ce que** la lame (38) et/ou la plaque de coupe (20) sont prévues avec un revêtement résistant à l'usure.

25. Instrument chirurgical selon la revendication 24 **caractérisé en ce que** le revêtement résistant à l'usure est ou contient du nitrure de titane (TiN).

26. Instrument chirurgical selon l'une quelconque des revendications précédentes, **caractérisé en ce qu'**une unité de codage coopérant avec une unité de décodage du dispositif de commande (92) est prévue sur la tige (18) pour coder la nature ou le type d'élément de coupe (36).

27. Instrument chirurgical selon la revendication 26, **caractérisé en ce que** l'unité de codage comprend au moins une saillie décollée de la tige (18).
